# EUROPEAN PATENT SPECIFICATION

(11) **EP 1 682 215 B1**
(45) Date of publication and mention of the grant of the patent: **24.12.2008**
(21) Application number: 04796207.1
(22) Date of filing: 22.10.2004
(51) Int. Cl.: A61N 1/05, A61B 17/04

(54) **MYOCARDIAL LEAD ATTACHMENT SYSTEM**
MYOKARDIALES LEITUNGSBEFESTIGUNGSSYSTEM
SYSTEME DE FIXATION DE DERIVATION MYOCARDIQUE

(30) Priority: 24.10.2003 US 514146 P; 24.10.2003 US 514037 P; 24.10.2003 US 514038 P; 24.10.2003 US 514042 P; 24.10.2003 US 514039 P; 27.10.2003 US 514713 P; 27.10.2003 US 514714 P; 27.10.2003 US 514665 P
(43) Date of publication of application: 26.07.2006
(73) Proprietor: CARDIAC PACEMAKERS, INC., St. Paul, Minnesota 55112-5798 (US)
(72) Inventor: COE, M., Sean, Plymouth, Minnesota 55441 (US); HEIL, Ronald W., Jr., Roseville, MN 55113 (US); KELLEY, Peter, T., Buffalo, Minnesota 55313 (US); PALME, Donald, F., Princeton, Minnesota 55371 (US); SHIROFF, Jason, Alan, Shoreview, Minnesota 55126 (US); WESTLUND, Randy, W., River Falls, wi 54022 (US); YINGLING, David, B., Stillwater, mn 55082 (US); GRESL, Charles, San Francisco, CA 94131 (US)
(74) Representative: Pfenning, Meinig & Partner GbR
(86) International application number: PCT/US2004/035172
(87) International publication number: WO 2005/039691

(56) References cited:
- EP-A- 1 025 802
- GB-A- 2 025 236
- US-A- 5 318 543
- US-A- 5 693 081
- US-A1- 2001 039 436
- US-A1- 2002 013 571
- US-B1- 6 473 654

## Description

### FIELD OF THE INVENTION

This invention relates generally to implantable lead assemblies for stimulating and/or sensing electrical signals in muscle tissue. More particularly, it relates to myocardially-implanted leads for cardiac stimulation and systems for anchoring the leads.

### BACKGROUND

Cardiac rhythm management systems are used to treat heart arrhythmias. Pacemaker systems are commonly implanted in patients to treat bradycardia (i.e., abnormally slow heart rate). A pacemaker system includes an implantable pulse generator and leads, which form the electrical connection between the implantable pulse generator and the heart. An implantable cardioverter defibrillator ("ICD") is used to treat tachycardia (i.e., abnormally rapid heart rate). An ICD also includes a pulse generator and leads that deliver electrical energy to the heart.

The leads coupling the pulse generator to the cardiac muscle are commonly used for delivering an electrical pulse to the cardiac muscle, for sensing electrical signals produced in the cardiac muscle, or for both delivering and sensing. The leads are susceptible to categorization according to the type of connection they form with the heart. An endocardial lead includes at least one electrode at or near its distal tip adapted to contact the endocardium (*i.e.*, the tissue lining the inside of the heart). An epicardial lead includes at least one electrode at or near its distal tip adapted to contact the epicardium (i.e., the tissue lining the outside of the heart). Finally, a myocardial lead includes at least one electrode at or near its distal tip inserted into the heart muscle or myocardium (i.e., the muscle sandwiched between the endocardium and epicardium). Some leads have multiple spaced apart distal electrodes at differing polarities and are known as bipolar type leads. The spacing between the electrodes can affect lead performance and the quality of the electrical signal transmitted or sensed through the heart tissue.

The lead typically consists of a flexible conductor surrounded by an insulating tube or sheath that extends from the electrode at the distal end to a connector pin at the proximal end. Endocardial leads are typically delivered transvenously to the right atrium or ventricle and commonly employ tines at a distal end for engaging the trabeculae.

The treatment of congestive heart failure ("CHF"), however, often requires left ventricular stimulation either alone or in conjunction with right ventricular stimulation. For example, cardiac resynchronization therapy ("CRT") (also commonly referred to as biventricular pacing) is an emerging treatment for heart failure, which requires stimulation of both the right and the left ventricle to increase cardiac output. Left ventricular stimulation requires placement of a lead in or on the left ventricle near the apex of the heart. One technique for left ventricular lead placement is to expose the heart by way of a thoracotomy. The lead is then positioned so that one or more electrodes contact the epicardium or are embedded in the myocardium. Another method is to advance an epicardial lead endovenously into the coronary sinus and then advance the lead through a lateral vein of the left ventricle. The electrodes are positioned to contact the epicardial surface of the left ventricle.

The left ventricle beats forcefully as it pumps oxygenated blood throughout the body. Repetitive beating of the heart, in combination with patient movement, can sometimes dislodge the lead from the myocardium The electrodes may lose contact with the heart muscle, or spacing between electrodes may alter over time.
WO 2005/028023 (document according to Articles 54(3) and 54(4) EPC) relates to a medical electrical lead anchoring wherein a lead body of a medical electrical lead includes a distal end, a first elongated insulated conductor extending toward the distal end and a first electrode coupled to the conductor. A second elongated insulated conductor includes a first portion extending within the lead body to the distal end and a second portion extending distally from the distal end of the lead body where it is terminated by an issue anchor on which a second electrode is mounted. The issue anchor includes a surface for receiving a push force from an insertion tool, which is adapted to insert the anchor within a segment of tissue such that the first electrode of the lead body is in close proximity to the segment of issue.
WO-2004/091716 (document according to Articles 54(3) and ⁵4(4) EPC) relates to a cardiac electrode anchoring system wherein a cardiac electrode arrangement has one or several heart electrodes arranged on the exterior surface of a heart or attached to the heart from the outside and/or arranged with a pole in the cardiac tissue. The electrodes, which can be fixed in the operating position by an anchor, run to an implanted heart pacemaker. For positioning and fixing the anchor, a tool is used that is designed as a rod or stylet and acts on an attachment site of the anchor, whereby the anchor can be pushed into or through the myocardium. The anchor is, at the same time, attached to a tension element or thread, over which the electrode, which has an inner guide channel that accommodates the tension element or thread, can be moved in a fitted and controlled manner. In the operating position, the electrode can be connected to the tension element or thread located in the guide channel.

There is a need for an improved myocardial pacing lead suitable for chronic implantation and a minimally invasive delivery system and method for implanting such a lead.

### BRIEF SUMMARY OF THE INVENTION

This object is obtained by a myocardial lead attachment system in accordance with the technical teachings of the accompanying claims.

While multiple embodiments are disclosed, still other embodiments of the present invention will become apparent to those skilled in the art from the following detailed description, which shows and describes illustrative embodiments of the invention. As will be realized, the invention is capable of modifications in various obvious aspects, all without departing from the spirit and scope of the present invention. Accordingly, the drawings and detailed description are to be regarded as illustrative in nature and not restrictive.

### BRIEF DESCRIPTION OF THE DRAWINGS

FIG. 1 is a schematic view of a portion of the vasculature and a myocardial lead attachment system according to one embodiment of the present invention.

FIG. 2 is a side view of a delivery instrument for use in conjunction with the myocardial lead attachment system of FIG. 1 according to one embodiment of the present invention.

FIG. 3A is a side view of a distal portion of the delivery instrument of FIG. 2 according to one embodiment of the present invention.

FIG. 3B is a side view of the distal portion of the delivery instrument of FIG. 2 according to another embodiment of the present invention.

FIG. 3C shows a side sectional view of the distal portion of the delivery instrument shown in FIG. 3A.

FIG. 3D is a perspective view of the distal portion of the delivery instrument shown in FIG. 3A.

FIG. 4A is a sectional view of a proximal portion of the delivery instrument of FIG. 2 according to one embodiment of the present invention.

FIG. 4B is a perspective view of a distal portion of the delivery instrument of FIG. 2 according to one embodiment of the present invention.

FIG. 4C is a partial sectional view of a distal portion of the delivery instrument of FIG. 2 according to another embodiment of the present invention.

FIG. 5A is a side view of an adjustable length delivery instrument in an extended position according to one embodiment of the present invention.

FIG. 5B is a side view of the delivery instrument of FIG. 5A in a retracted position.

FIG. 6 is a sectional side view of a portion of an adjustable length delivery instrument according to another embodiment of the present invention.

FIG. 7 is a sectional view of a distal portion of a delivery instrument for use in conjunction with the myocardial lead attachment system of FIG. 1 according to one embodiment of the present invention.

FIG. 8 is a sectional view of a distal portion of a delivery instrument for use in conjunction with the myocardial lead attachment system of FIG. 1 according to another embodiment of the present invention.

FIG. 9 is a sectional view of a distal portion of a delivery instrument for use in conjunction with the myocardial lead attachment system of FIG. 1 according to still another embodiment of the present invention.

FIG. 10 is a sectional side view of a distal portion of a delivery instrument for use in conjunction with the myocardial lead attachment system of FIG. 1 according to yet another embodiment of the present invention.

FIG. 11 is a sectional side view of a distal portion of a delivery instrument for use in conjunction with the myocardial lead attachment system of FIG. 1 according to another embodiment of the present invention.

FIG. 12 is a side view of a distal portion of a delivery instrument for use in conjunction with the myocardial lead attachment system of FIG. 1 according to still another embodiment of the present invention.

FIG. 13 is a side view of a delivery instrument for use in conjunction with the myocardial lead attachment system of FIG. 1 according to another embodiment of the present invention.

FIG. 14 is a side view of a delivery instrument for use in conjunction with the myocardial lead attachment system of FIG. 1 according to another embodiment of the present invention.

FIG. 15A is a sectional view of the heart schematically illustrating the use of a delivery instrument in conjunction with a myocardial lead attachment system in an epicardial-epicardial procedure according to one embodiment of the present invention.

FIG. 15B is a sectional view of the heart schematically illustrating the use of a delivery instrument in conjunction with a myocardial lead attachment system in an epicardial-endocardial configuration according to one embodiment of the present invention.

FIG. 15C is a sectional view of a portion of the heart schematically illustrating the use of a delivery instrument in conjunction with a myocardial lead attachment system in an intra-myocardial procedure according to another embodiment of the present invention.

FIG, 15D is a sectional view of a portion of the heart schematically illustrating the use of a delivery instrument in conjunction with a myocardial lead attachment system in a pericardial-pericardial procedure according to yet another embodiment of the present invention.

FIG. 16A is a sectional view of a portion of the heart and a schematically illustrated distal portion of a myocardial lead attachment system having a unipolar electrode arrangement according to one embodiment of the present invention.

FIG. 16B is a sectional view of a portion of the heart and a schematically illustrated distal portion of a myocardial lead attachment system having a pseudo-unipolar electrode arrangement according to one embodiment of the present invention.

FIG. 16C is a sectional view of a portion of the heart and a schematically illustrated distal portion of a myocardial lead attachment system having a pseudo-unipolar electrode arrangement according to yet another embodiment of the present invention.

FIG. 16D is a sectional view of a portion of the heart and a schematically illustrated distal portion of a myocardial lead attachment system having a pseudo-unipolar electrode arrangement according to still another embodiment of the present invention.

FIG. 16E is a sectional view of a portion of the heart and a schematically illustrated distal portion of a myocardial lead attachment system having a bipolar electrode arrangement according to another embodiment of the present invention.

FIG. 17 is a sectional view of the distal portion of the myocardial lead attachment system of FIG. 1 according to one embodiment of the present invention.

FIG. 18A is a sectional view of a distal portion of a myocardial lead attachment system according to another embodiment of the present invention.

FIG. 18B is a side view of the tether of FIG. 18A.

FIG. 18C is a perspective view of the lock of FIG. 18A.

FIG. 19 is a sectional view of a distal portion of the myocardial lead attachment system of FIG. 18A implanted in the myocardium according to another embodiment of the present invention.

FIG. 20 is a sectional view of the distal portion of the myocardial lead attachment system of FIG. 18A implanted in the myocardium according to yet another embodiment of the present invention.

FIG. 21 is a sectional view of a distal portion of a myocardial lead attachment system implanted in the myocardium according to yet still another embodiment of the present invention.

FIG. 22A is a sectional view of a proximal portion of a myocardial lead attachment system detailing a proximal lock in a first configuration according to one embodiment of the present invention.

FIG. 22B is a sectional view of the myocardial lead attachment system of FIG. 22A showing the proximal lock in a second configuration.

FIG. 23A is a sectional view of a proximal portion of a myocardial lead attachment system detailing a proximal lock according to one embodiment of the present invention.

FIG. 23B is a sectional view of a proximal portion of a myocardial lead attachment system detailing a proximal lock according to another embodiment of the present invention.

FIG. 23C is a sectional view of a proximal portion of a myocardial lead attachment system detailing a proximal lock according to still another embodiment of the present invention.

FIG. 24 is a perspective view of a proximal portion of a myocardial lead attachment system according to another embodiment of the present invention.

FIG. 25 is a side view of a distal portion of a myocardial lead attachment system according to one embodiment of the present invention.

FIG. 26 is a side view of a distal portion of a myocardial lead attachment system according to another embodiment of the present invention.

FIG. 27 is a side view of a distal portion of a myocardial lead attachment system according to yet another embodiment of the present invention.

FIG. 28 is a side sectional view of a distal portion of a myocardial lead attachment system including a rapidly dissolvable coating according to another embodiment of the present invention.

FIG. 29A is a side sectional view of the anchor mechanism of FIG. 28 including a rapidly dissolvable coating according to another embodiment of the present invention.

FIG. 29B is a side sectional view of the anchor mechanism of FIG. 28 according to yet another embodiment of the present invention.

FIG. 30 is a side sectional view of a distal portion of the myocardial lead attachment and pacing system of FIG. 1.

FIG. 31 is a side sectional view of the fixation mechanism shown in FIG. 30 according to another embodiment of the present invention.

FIG. 32 is a side sectional view of the fixation mechanism shown in FIG. 30 according to another embodiment of the present invention.

FIG. 33A is a side sectional view of the fixation mechanism shown in FIG. 30 including a surface feature according to another embodiment of the present invention.

FIG. 33B is a side sectional view of the fixation mechanism of FIG. 33A including a dissolvable coating according to one embodiment of the present invention.

FIG. 33C is a side sectional view of the fixation mechanism of FIG. 33A including a dissolvable coating according to another embodiment of the present invention.

FIG. 34 is a side sectional view of a distal portion of a myocardial lead attachment system according to another embodiment of the present invention.

FIG. 35A is a partial sectional view of portion of the vasculature schematically illustrating a myocardial lead attachment system in an epicardial-epicardial configuration.

FIG. 35B is a partial sectional view of portion of the vasculature schematically illustrating a myocardial lead attachment system in an epicardial-endocardial configuration.

FIG. 35C is a partial sectional view of portion of the vasculature schematically illustrating a myocardial lead attachment system in an intramyocardial configuration.

FIG. 35D is a partial sectional view of portion of the vasculature schematically illustrating a myocardial lead attachment system in a pericardial-pericardial configuration.

FIG. 36 is a side sectional view of a distal portion of the myocardial lead attachment system of FIG. 1 in accordance with one embodiment of the present invention.

FIG. 37 is a side sectional view of a distal portion of the myocardial lead attachment system of FIG. 1 in accordance with another embodiment of the present invention.

FIG. 38 is a side sectional view of a distal portion of the myocardial lead attachment system of FIG. 1 in accordance with yet another embodiment of the present invention.

FIG. 39 is a side sectional view of a distal portion of the myocardial lead attachment system of FIG. 1 in accordance with still another embodiment of the present invention.

FIG. 40A is a side sectional view of a distal portion of the myocardial lead attachment system of FIG. 1 in accordance with another embodiment of the present invention.

FIG. 40B is a side sectional view of the attachment system of FIG. 40A following removal of the tip.

FIG. 41 is a perspective view of a distal portion of the myocardial lead attachment system of FIG. 1 in accordance with yet another embodiment of the present invention.

### DETAILED DESCRIPTI,ON

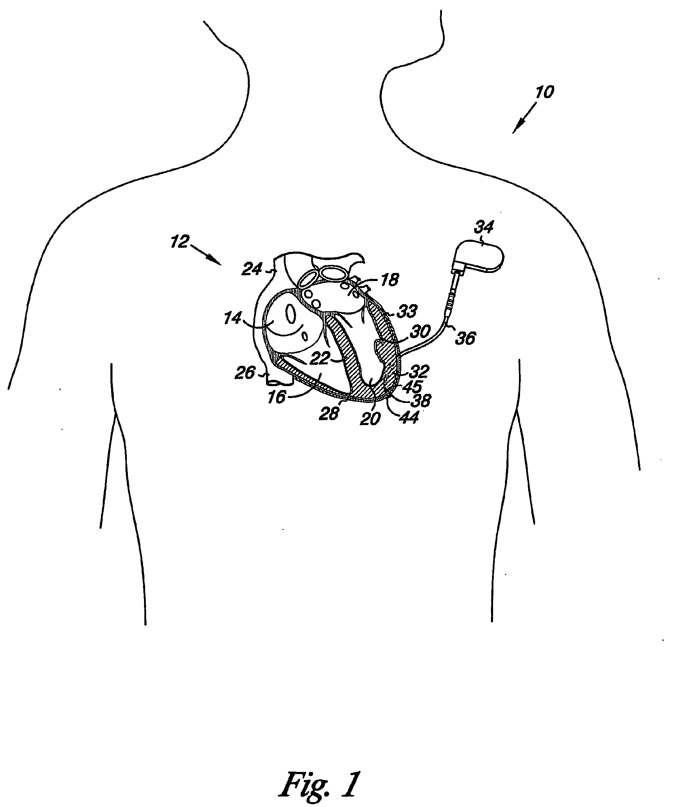

FIG. 1 shows a myocardial lead attachment system 10 deployed in a human heart 12 according to one embodiment of the present invention. The heart 12 includes a right atrium 14 and a right ventricle 16 separated from a left atrium 18 and a left ventricle 20 by a septum 22. During normal operation of the heart 12, deoxygenated blood is fed into the right atrium 14 through the superior vena cava 24 and the inferior vena cava 26. The deoxygenated blood flows from the right atrium 14 into the right ventricle 16. The deoxygenated blood is pumped from the right ventricle 16 into the lungs, where the blood is re-oxygenated. From the lungs the oxygenated blood flows into the left atrium 18, then into the left ventricle 20. The left ventricle 20 beats forcefully to pump the oxygenated blood throughout the body.

The outer walls of the heart 12 are lined with a tissue known as the epicardium 28. The inner walls of the heart are lined with a tissue known as the endocardium 30. The heart muscle, or myocardium 32, is sandwiched between the endocardium 30 and the epicardium 28. A tough outer pericardial sac 33 surrounds the heart 12.

The myocardial lead attachment system 10 includes a pulse generator 34 coupled to a myocardial lead 36. The pulse generator 34 is typically implanted in a pocket formed underneath the skin of the patient's chest or abdominal region. The lead 36 extends from the pulse generator 34 to the heart 12 and is implanted in the myocardium 32 near an apex 38 of the heart 12. The lead 36 delivers electrical signals from the pulse generator 34 to an electrode located at or near a distal tip to accomplish pacing of the heart 12 (not shown in FIG. 1). An anchor mechanism 44 is coupled to the lead 36 via a tether 45 to secure the lead 36 to the heart 12 and to retain the electrode in a chosen location.

According to one embodiment, and generally shown in the following figures, the anchor mechanism 44 is an elongated T-bar, in which the tether 45 is coupled to the anchor mechanism 44 between its opposite ends and preferably at a mid-point to form the "T". According to other embodiments, the anchor mechanism 44 has any other shape suitable for forming an anchor against the heart 12 to retain the lead 36 in the desired location. An important feature of the anchor mechanism 44 is that its shape is configured to engage the heart tissue following implantation through a tract (not visible in FIG. 1) in the heart 12 to reduce the likelihood of re-entry of the anchor mechanism 44 into the tract. According to one embodiment, the length of the anchor mechanism 44 is greater than the diameter of the tract. The "T" configuration is such that tension exerted on the mid-point of the anchor mechanism 44 via the tether 45 tends to position the anchor mechanism 44 cross-wise over the tract, preventing the anchor mechanism 44 from sliding proximally through the tract. The lead 36 is then advanced over the tether 45 into the myocardium 32. The anchor mechanism 44 resists the tensioning force exerted on the tether 45 such that the lead 36 may be easily advanced through the tract.

Placement of the lead 36 and anchor mechanism 44 of FIG. 1 may be accomplished by exposing a portion of the heart 12, for example by way of a stemotomy, thoracotomy or mini-thoracotomy. According to other embodiments, the heart 12 may be accessed via an endoscopic procedure according to known methods. Although shown implanted near the apex 38, the lead 36 may be implanted in the heart 12 anywhere pacing therapy is needed. The anchor mechanism 44 is delivered to the heart 12 with a delivery instrument according to any of the following embodiments.

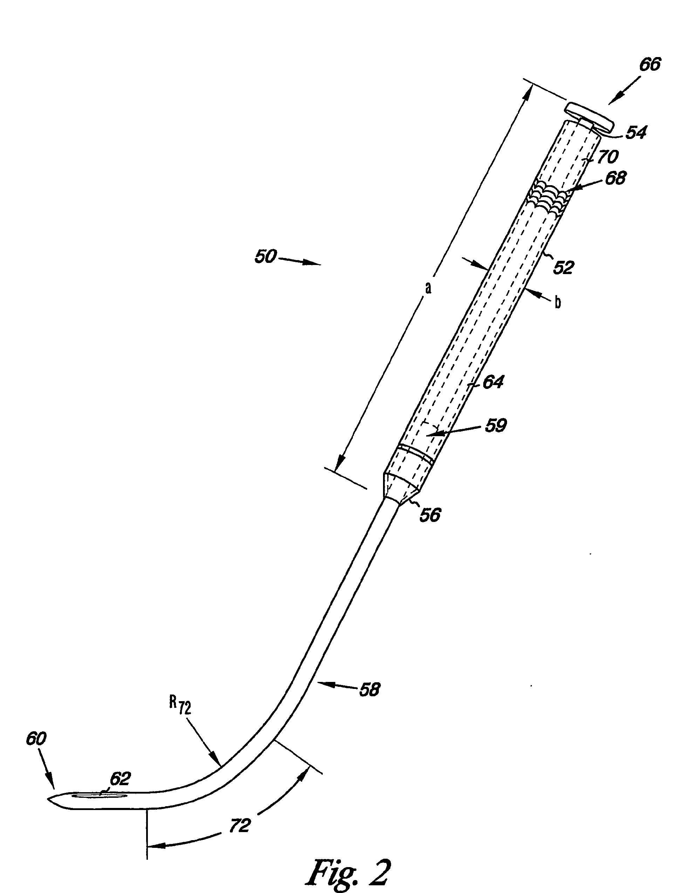

FIG. 2 shows a delivery instrument 50 according to one embodiment of the present invention. The delivery instrument 50 includes a needle portion 58 having a distal tip 60 and a nest 62. The nest 62 is sized to receive at least a portion of the anchor mechanism 44 (not visible in FIG. 2). The delivery instrument 50 further includes an ejection mechanism 66 coupled to an actuator (not visible in FIG. 2) for ejecting the anchor mechanism 44 from the nest 62.

The delivery instrument 50 includes a handle 52 having a proximal end 54 and a distal end 56. The needle portion 58 is coupled to the handle 52 and extends from the distal end 56. The needle portion 58 is of a hypotube construction with an internal lumen 59. The handle 52 has an internal lumen 64 continuous with the needle lumen 59. The ejection mechanism 66 is located on the handle 52 and is operable within the internal lumen 64. According to other embodiments, the ejection mechanism 66 is located elsewhere on the handle 52.

The handle 52 is sized with a length a and diameter b for easy grasping and manipulation. According to one embodiment of the present invention, a diameter b of about 5 mm provides a mechanical advantage allowing stable control over the direction of the distal needle tip 60. Further, a diameter b of about 5 mm can be used within a standard 7 mm or larger thoracic port, which is commonly used in minimally-invasive thoracoscopic procedures to access the heart 12.

An outer surface 70 of the handle 52 includes a surface feature 68. Surface feature 68 may include bumps, ribs, or bulging features on the outer surface 70 chosen to enhance the friction between the handle 58 and the surgeon's hand. Surface feature 68 advantageously reduces slippage between the surgeon's hand and the handle 52 while in wet, slippery environments. The handle 52 can be made of any sterilizable metal or polymeric material, including stainless steel, polypropylene, and polyurethane.

The shape of the distal needle portion 58 is configured to facilitate access to the heart 12 via thoracotomy or other procedures, such as those mentioned above. According to one embodiment of the present invention, the needle portion 58 is shaped with a curved portion 72. The shape of the curved portion 72 will be dictated in part by patient anatomy (e.g., overall heart size, peculiarities in geometry due to dilation, epicardial fat, infarct, and vessels), location of the thoracotomy (posterior or anterior to an imaginary mid-axillary line bisecting the heart 12), and size of the incision into the heart tissue. According to one embodiment, the curved portion 72 has a radius of curvature R₇₂ of about 22 mm, a shape intended to be approximately what the physician would need for implanting the lead 36 via a thoracotomy. According to other embodiments, the radius of curvature R₇₂ of curved portion 72 is from about 10 to about 35 mm (not shown). According to other embodiments, the curved portion 72 is constructed of multiple curved segments, or is straight (not shown).

According to one embodiment, the needle portion 58 is constructed of stainless steel. The curved portion 72 may be formed according to a variety of methods. The curved portion 72 may be formed by mechanically bending the needle portion 58. Alternately, the needle portion 58 is heat-set so that the needle portion 58, or a smaller portion thereof, is malleable. A chosen curvature can be imparted to the needle portion 58 contemporaneous with the implantation procedure according to the surgeon's needs.

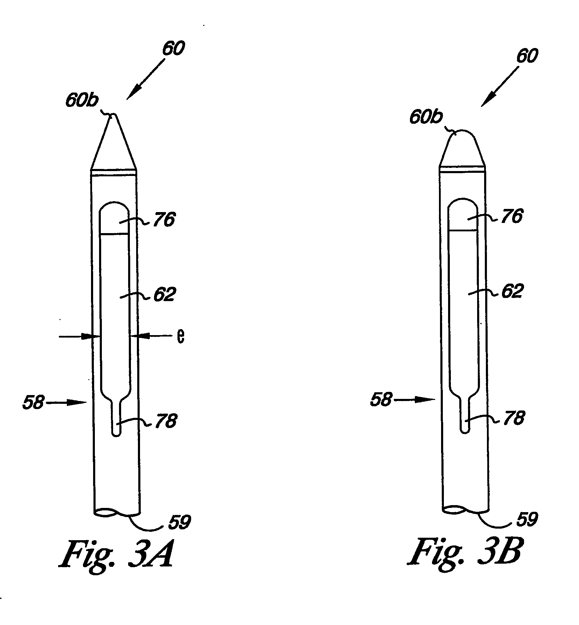

FIG. 3A shows a portion of the needle portion 58 according to one embodiment of the present invention. The tip 60 and nest 62 are shown as previously described. A distal end 60b of the tip 60 is configured to minimize trauma to the myocardium 32 during insertion. This is accomplished by dissecting the myocardium 32 as opposed to cutting the myocardium 32. According to one embodiment, the tip 60 is generally conical in shape, terminating at a slightly rounded point.

FIG. 3B shows another embodiment in which the distal end 60b of the tip 60 is more rounded or blunt than the embodiment shown in FIG. 3A. A rounded or blunted tip 60 is understood to be less traumatic and less likely to damage coronary vessels when passing through the myocardium 32. According to one embodiment, the tip 60 is generally isodiametric. An isodiametric configuration generally exerts force equally on the myocardial tissue 32 about the tip 60 when passing through tissue. A uniform force distribution reduces excessive force on the myocardial tissue 32 in directions other than along the axis of dissection. According to other embodiments, the tip 60 is provided with a sharp cutting edge.

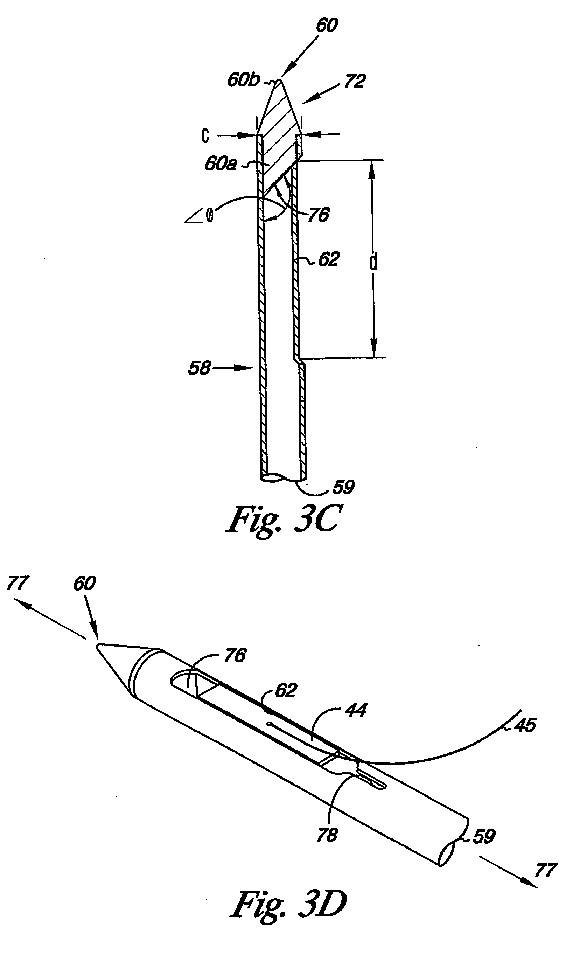

The nest 62 is sized with a length d and a width e chosen such that the nest 62 retains the anchor mechanism 44 when the needle portion 58 is advanced through the myocardium 32 (anchor mechanism 44 not visible in FIGS. 3A and 3B - see FIG. 3D). According to one embodiment, the nest 62 is an elongated slot in the needle portion 58 opening to the needle lumen 59. The nest 62 further includes a tether slot 78 located near its proximal end. The tether slot 78 is adapted to accept the tether 45 when the needle portion 58 is advanced through the myocardium 32 (See FIG. 3D).

FIG. 3C shows the needle portion 58 of FIG. 3A. A proximal end 60a of the tip 60 is sized with a diameter c chosen to mate with the needle lumen 59. The nest 62 includes a distal angled face or ramp 76 facing the interior of the nest 62. The ramp 76 is disposed at an angle θ relative to a longitudinal axis 77 of the needle 58.

FIG. 3D shows the anchor mechanism 44 received in the nest 62. The anchor mechanism 44 is placed in the slot 62 and slid proximally into the needle lumen 59. The tether 45 is received in the tether slot 78, permitting the anchor mechanism 44 to be slid further into the lumen 59. The ramp 76 is inclined away from the anchor mechanism 44 at the angle θ such that distal sliding of the anchor mechanism 44 along the axis 77 causes the anchor mechanism 44 to ride up the ramp 76 and out of the nest 62.

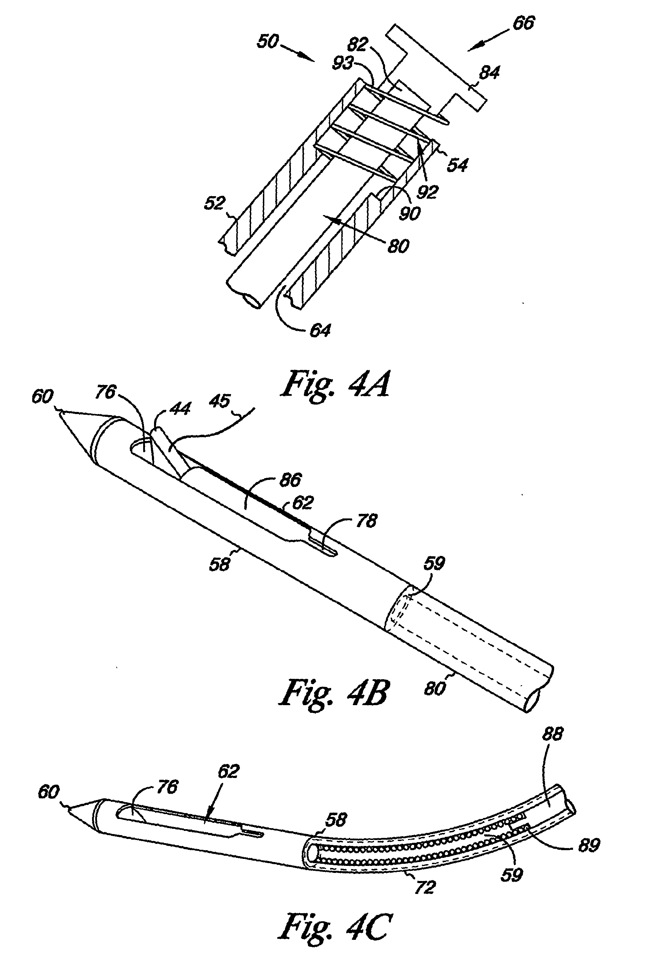

FIG. 4A shows a proximal portion of the handle 52 and FIG. 4B shows a distal portion of the needle portion 58, both detailing the ejection mechanism 66. The ejection mechanism 66 includes an elongated member 80 extending from the proximal end 54 of the handle 52, through the handle lumen 64 and needle lumen 59, and terminating proximal to the nest 62. A proximal end 82 of the elongated member 80 is operationally coupled to an actuator 84. According to one embodiment, the actuator 84 is a depressible button operable to slide the elongated member 80 distally from a neutral or retracted position to an advanced position. FIG. 4B shows the elongated member 80 in an advanced position such that a distal end 86 of the elongated member 80 has engaged the anchor mechanism 44 and is pushing the anchor mechanism 44 distally within the nest 62. Doing so forces the anchor mechanism 44 to slide distally along the ramp 76 and out of the nest 62.

According to one embodiment, shown in FIGS. 4A and 4B, the elongated member 80 is a solid tubular member sufficiently flexible to navigate the curved portion 72 (see FIG. 2) of the needle portion 58. According to another embodiment, as shown in FIG. 4C, the elongated member 80 is constructed of a combination of a solid member 88 and a helically-wound member 89. The solid member 88 extends through the needle lumen 59 to the curved portion 72 and transfers axial force to the distally positioned helical member 89, which extends through the needle lumen 59 at the curved portion 72. The helical member 89 is sufficiently flexible to navigate the curved portion 72, while the solid member 88 may be rigid. According to other embodiments, the elongated member 80 is a rigid member. Elongated member 80 may be constructed of a polymer or metal.

Returning to FIG. 4A, the handle lumen 64 is formed with a ledge 90 forming a mechanical stop. The ledge 90 blocks actuation of the actuator 84 (i.e. further depression of the button), and prevents over-advancement of the elongated member 80. A biasing mechanism 92, including a resilient member 93, such as a spring, is interposed between the actuator 84 and the handle 52. The biasing mechanism 92 biases the actuator 84 to a neutral position in which the elongated member 80 is retracted.

According to one embodiment, the ejection mechanism 66 and actuator 84 are operable to advance the elongated member 80 to one of a plurality of pyre-set positions. According to another embodiment, the present invention is an automatic or semi-automatic delivery instrument. In this embodiment a lever mechanism, or trigger and spring mechanism, drives the needle portion 58 from a retracted position into the myocardium 32 in a prescribed path, ejects the anchor mechanism 44 and retracts the needle portion 58 to its original position.

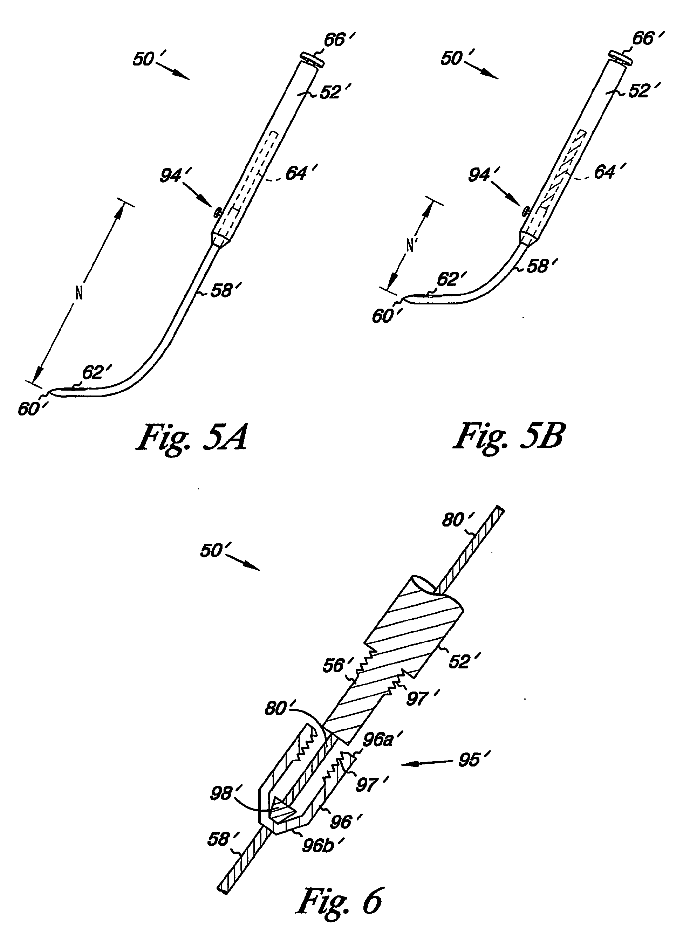

FIGS. 5A and 5B show a delivery instrument 50' which is generally similar to the delivery instrument 50 shown FIG. 2. The length of the needle portion 58' of the instrument 50' extending from the handle 52' is adjustable by sliding portions of the needle portion 58' into the handle lumen 64'. In one embodiment, needle portion 58' is retained in a chosen position within the handle lumen 64' via a keeper mechanism, such as a set screw 94', extending through the handle 52'. If the physician desires more needle length, N, as shown in FIG. 5A, either for patients with thick thoracic walls requiring a deeper reach to the epicardium 28 or for thoracoscopic procedures, he may adjust accordingly. If he requires less length N', as shown in FIG. 5B, the needle portion 58' may be retracted into the lumen 64'.

FIG. 6 shows another embodiment of the adjustable length delivery instrument 50' in which the needle portion 58' is adjustably coupled to the handle 52' via a collet mechanism 95'. According to one embodiment, the needle portion 58' is provided with a sleeve 96' having an open end 96a' facing proximally and a distal end 96b' coupled to the needle portion 58' via guide member 98'. The sleeve 96' is sized to receive the distal end 56' of the handle 52'. The sleeve 96' and the distal end 56' of the needle 52' are provided with complementary threads 97'. Rotation of the sleeve 96' about the handle threads 98' advances the sleeve 96' onto the handle 52', shortening the effective length of the needle portion 58'. Reverse rotation advances the needle portion 58' distally from the handle 52', increasing the effective length of the needle 58'. According to other embodiments, the needle 58' and handle 52' may be provided with complementary teeth or other means for providing an adjustable length coupling.

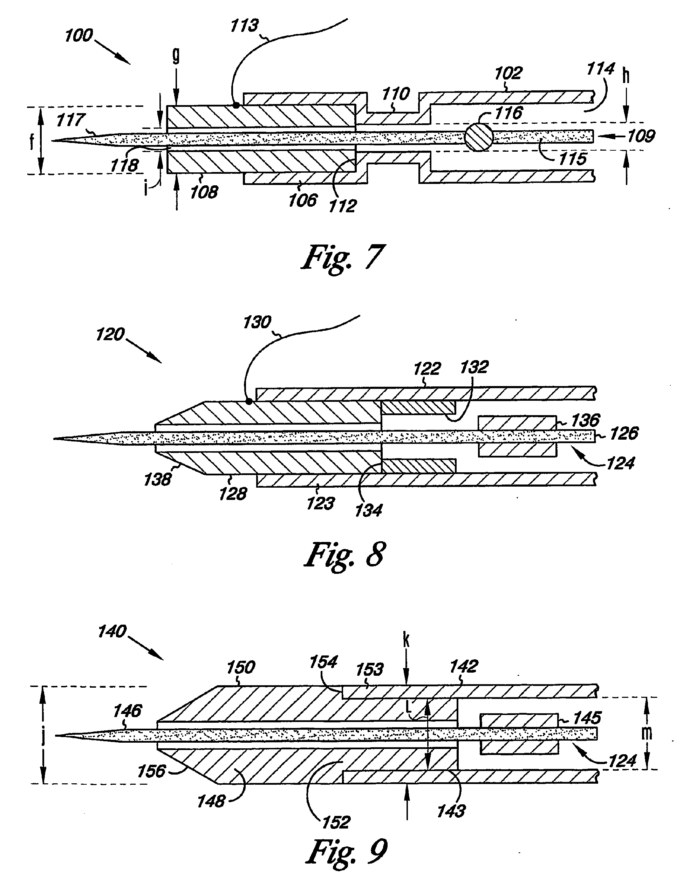

FIG. 7 shows a delivery instrument 100 for use in conjunction with the myocardial lead attachment system 10 of FIG. 1 according to another embodiment of the present invention. The instrument 100 includes a tube or needle 102 having a nest 106 for receiving an anchor mechanism 108, and an ejection mechanism 109 for ejecting the anchor mechanism 108 from the nest 106. A proximal portion of the needle 102 may be attached to a handle to facilitate manipulation (not shown).

According to the present embodiment, the nest 106 is an open sleeve at a distal end of the needle 102. The anchor mechanism 108 is received in the nest 106 and protrudes outwardly from the nest 106. The needle 102 includes a crimp or detent 110 near to and proximal to the nest 106 for contacting a proximal or trailing edge 112 of the anchor mechanism 108. An inside diameter f of the nest 106 is slightly greater than an outside diameter g of the anchor mechanism 108, such that the anchor mechanism 108 can readily release from the nest 106 upon application of a relatively small force on the anchor mechanism 108 directed distally from the needle 102. The anchor mechanism 108 is coupled to a tether 113 as previously described. The needle 102 is further provided with a central lumen 114 extending therethrough.

The ejection mechanism 109 is a stylet 115 coupled to an ejection plug 116. The stylet 115 has a sharpened distal tip 117 shaped to effectively dissect the tissue of the myocardium 32. According to other embodiments, the tip 117 is blunt or rounded to reduce trauma to the myocardium 32. The stylet 115 extends through the needle lumen 114 and through a bore 118 extending longitudinally through the anchor mechanism 108. The ejector plug 116 is spherical and is sized and shaped smaller than an inside diameter h of the needle 102 at the crimp 110 so as to pass through the crimp 110 but larger than an inside diameter / of the bore 118 in the anchor mechanism 108 so as to contact the trailing edge 112 of the anchor mechanism 108. According to other embodiments, the ejector plug 116 has a cylindrical or other shape. According to still other embodiments, the ejector plug 116 is not formed integrally with the stylet 115 but rather is a separate member such as a clip that is affixed to the stylet 115.

During implantation, the anchor mechanism 108 is received in the nest 106. The tip 117 of the stylet 115 extends through the bore 118 in the anchor mechanism 108 and protrudes from the bore 118. The tube 102 and the stylet 115 are used to guide the anchor mechanism 108 through the myocardium 32. The crimp 110 contacts the trailing edge 112 of the anchor mechanism 108 to force the anchor mechanism 108 through the myocardium 32. Once the anchor mechanism 108 emerges from the myocardium 32, the ejection mechanism 109 is actuated by advancing the stylet 115 distally with respect to the tube 102, such that the ejection plug 116 contacts the trailing end 112 of the anchor mechanism 108 and ejects the anchor mechanism 108 from the nest 106. This deploys the anchor mechanism 108 on the surface of the heart 12. The needle 102 and stylet 115 are then withdrawn.

According to one embodiment, the ejection mechanism 109 is coupled to an actuator as described with respect to the embodiment shown generally in FIGS. 2-6 to distally advance the stylet 106 (not shown). According to other embodiments, the ejection mechanism 109 is actuated by manually advancing the stylet 115 with respect to the needle 102.

FIG. 8 shows a distal portion of a delivery instrument 120 according to another embodiment of the present invention. As shown in FIG. 8, the instrument 120 includes many of the same components as the delivery instrument 100 of FIG. 7. The instrument 120 includes a tube or needle 122 having a nest 123, an ejection mechanism 124 including a stylet or guide wire 126 and an anchor mechanism 128 coupled to a tether 130. The instrument 120 does not include a crimp or detent 110 as described with respect to instrument 100 of FIG. 7, but instead includes a ledge 132 forming an internal stop in the needle 122 proximal to the nest 123. The ledge 132 contacts a trailing edge 134 of the anchor mechanism 128 to drive the anchor 128 forward during implantation. The outer profile of the needle 102 is smooth and continuous.

The stylet 126 is provided with an ejector plug 136 as described previously. According to the present embodiment, however, the ejector plug 136 is cylindrical in shape. According to the embodiment shown, the anchor mechanism 128 includes an angled or tapered leading edge 138. Tapered edge 138 facilitates dissection of the myocardium 32 during implantation. The leading edge 138 of the anchor mechanism 128 may have a blunt or rounded profile similar to the profile of the needle tip 60 described with reference to the embodiment shown in FIGS. 3A and 3B. The instrument 120 is used in a similar manner as described above with reference to the embodiment shown in FIG. 7.

FIG. 9 shows a sectional view of a distal portion of a stylet delivery instrument 140 according to another embodiment of the present invention. The instrument 140 includes many of the same components as the delivery instruments 100 and 120 of FIGS. 7 and 8. The instrument 140 includes a tube or needle 142 having a nest 143 and an ejection mechanism 144. The ejection mechanism 144 is a stylet or guide wire 146 having an ejector plug 145 as described previously. An anchor mechanism 148 having a stepped outside diameter is received in the nest 143. A distal portion 150 of the anchor mechanism 148 has a diameter j that is roughly equal to an outside diameter k of the needle 142. A proximal portion 152 of the anchor mechanism 148 has a diameter /slightly smaller than an inside diameter m of the needle 142, such that the anchor mechanism 148 can be inserted into the nest 143. In this embodiment, a leading edge 154 of the tube 142 serves to contact the anchor mechanism 148 and transmit axial force during implantation. In this embodiment, the anchor 148 may include either a blunt or a tapered leading edge 156 as described previously. The instrument 140 is used in the manner described above with reference to FIG. 7.

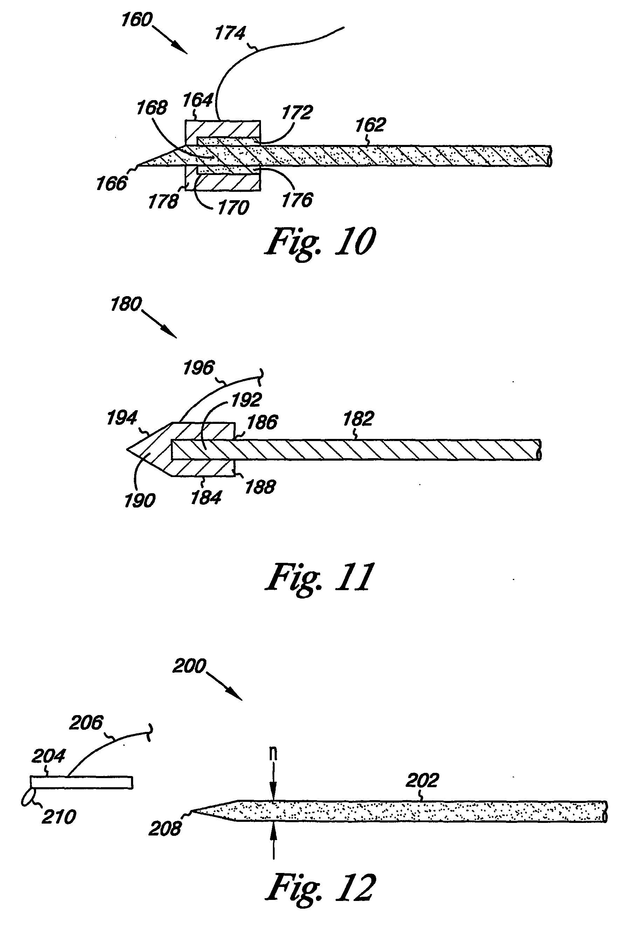

FIG. 10 shows a sectional view of a distal portion of a delivery instrument 160 according to another embodiment of the present invention. The instrument 160 includes a stylet 162 and an anchor mechanism 164. The stylet 162 includes a distal tip 166, which is shaped for efficient dissection of the myocardium 32. A distal end 168 of the stylet 162 is provided with a raised region 170 forming a mechanical stop. According to one embodiment, a tubular sleeve 172 is positioned about the stylet 162 to form the raised region 170. According to other embodiments the raised region 170 is integrally formed with the stylet 162. The anchor mechanism 164, which is coupled to a tether 174, has an inner bore 176 extending therethrough. An inner surface of the bore 176 includes a ledge 178. A distal end 168 of the stylet 162 is received in the bore 176, such that the tip 166 protrudes from the bore 176. The ledge 178 is adapted to mate with and contact the raised region 170 of the stylet 162 such that upon application of an axial force on the stylet 162, the raised region 170 contacts the ledge 178 and forces the anchor mechanism 164 to advance distally.

The instrument 160 is advanced through the heart 12, and the stylet 162 is manipulated into the heart 12 and position the anchor mechanism 164. When the anchor mechanism 164 has been properly positioned, the stylet 162 is withdrawn.

FIG. 11 shows a sectional view of a distal portion of a stylet delivery instrument 180 according to another embodiment of the present invention. The instrument 180 includes a stylet 182 and an anchor mechanism 184. The anchor mechanism 184 includes a blind bore 186 extending from a proximal end 188 of the anchor mechanism 184 but not all the way through to a distal end 190. The inner surface of the blind bore 186 has a diameter sized to receive a distal end 192 of the stylet 182. As shown, the anchor 184 has a tapered leading edge 194 to facilitate dissection of the myocardium 32. A tether 196 is attached to the anchor 184. The stylet 182 is used to advance the anchor mechanism 184 through the myocardium 32. When the anchor mechanism 184 is located in a chosen position, for example, on the endocardial surface 30, the stylet 182 is withdrawn.

FIG. 12 shows a side view of a stylet delivery instrument 200 according to another embodiment of the present invention. The instrument 200 includes a guide wire or stylet 202 and an anchor mechanism 204 coupled to a tether 206. The stylet 202 includes a tapered tip 208 shaped for efficient dissection of the myocardium 32. The anchor mechanism 204 is coupled to a catch mechanism 210 for receiving the stylet 202. The catch mechanism 210 is a ring-shaped member and may be formed from a looped region of the tether 206 or formed separately and attached to the anchor mechanism 204. According to one embodiment, the catch mechanism 210 is generally flexible. According to another embodiment, the catch mechanism 210 is rigid.

The catch mechanism 210 has an inner diameter smaller than an outside diameter n of the stylet 202, such that the stylet tip 208 can be inserted partially into the catch mechanism 210, but the stylet 202 cannot fully pass through the catch mechanism 210. The stylet tip 202 is inserted into the catch mechanism 210 so that the anchor 204 lies adjacent the length of the stylet 202. According to other embodiments, the stylet 202 is provided with a circumferential groove for receiving the catch mechanism 210 (not shown). The stylet 202 is inserted into the myocardium 32, drawing the anchor mechanism 204 along via the catch mechanism 210. Once the stylet tip 208 and the anchor mechanism 204 emerge from the myocardium 32, the stylet 202 is withdrawn, disengaging from the catch mechanism 210. Following implantation, the catch mechanism 210 may be removed or may be left in place.

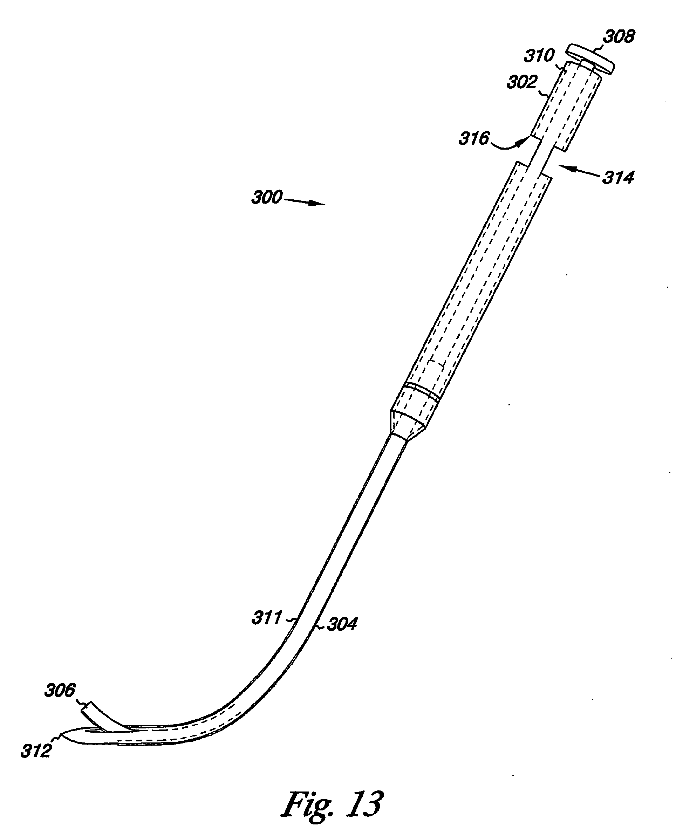

FIG. 13 shows a delivery instrument 300 in accordance with another embodiment of the present invention. The delivery instrument 300 includes many of the features of the delivery instrument 50 shown in FIGS. 2 - 6. In addition, the delivery instrument 300 may be used for identifying an appropriate or desired implant site. The delivery instrument 300 includes a handle 302, a needle 304, an anchor mechanism 306 and an ejection mechanism 308 as previously described. According to the present embodiment, the needle 304 is electrically conductive and extends nearly to a proximal end 310 of the handle 302. The needle 304 is electrically isolated or masked via a non-conductive insulating or dielectric material polymer sleeve 311 extending the length of the needle 304. According to one embodiment, the sleeve 311 is formed of a heat shrinkable polyimide. According to other embodiments, the sleeve 311 is formed of a conformal polymer such as parylene or ethylene-tetrafluoro ethylene. According to another embodiment, the needle 304 is coated with a layer of non-conductive polymer material.

The needle 304 is electrically exposed at a distal tip 312 and at a proximal region 314. The proximal exposed region 314 is accessible via a cut-out or window 316 formed in the handle 302. The needle 304 acts as an electrical conductor between the exposed proximal region 314 and the exposed distal tip 312. Alligator clips or other electrical connectors may be coupled to the needle 304 through the window 316 to electrically couple the exposed distal tip 312 to a pacing and sensing analyzer for performing sensing and pacing functions (not shown).

The exposed area at tip 312 is brought into contact with the epicardium 28 or myocardium 32 to perform sensing and pacing functions prior to ejection of the myocardial anchor 306. Additionally, acute therapeutic benefit at a particular site may be assessed using said embodiment. If acute benefit is unacceptable, the implant site may be changed prior to implanting the lead 36.

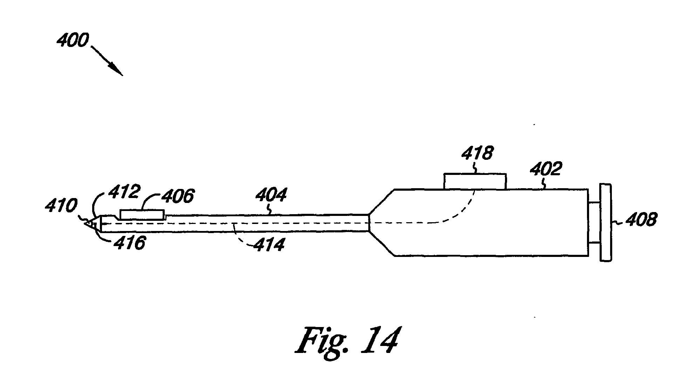

FIG. 14 shows a delivery instrument 400 according to another embodiment of the present invention. The delivery instrument 400 includes a handle 402 coupled to a needle 404, an anchor mechanism 406 and an ejection mechanism 408 as previously described with respect to various embodiments. In addition, the delivery instrument 400 may be used for identifying an appropriate or desired implant site. According to one embodiment, this is accomplished by electrically isolating the needle region 404 except for a small exposed or electrically active area 410 on the conical tip 412. The needle region 404 may be electrically isolated according to the techniques described with respect to the previously described embodiment shown in FIG. 13. A conductive wire 414 with a terminal end 416 in electrical communication with the electrically active area 410 is located in the handle 402. The wire 414 is connected to a pacing and sensing analyzer 418. The electrically active area 410 is brought into contact with the epicardium 28 or myocardium 32 to perform sensing and pacing as previously described.

The systems shown in FIGS. 2-14 are amenable to minimally-invasive introduction into the heart 12, such as by known thoracoscopic, mini-thoracoscopic or endoscopic techniques. In one embodiment employing an endoscopic technique, a small subxiphoid incision is made to gain visualization of the apex 38 of the heart 12. The pericardium 33 is perforated to gain access to the epicardial surface 28. An endoscopic probe having an open working channel is then introduced into the chest. The endoscopic probe includes common features such as a light tube, fiber optic visualization, and means to accomplish distal deflections of the tube or needle when formed of a malleable material as described above.

A target region of the heart 12 is approached and advanced sensing probes may be used to assess the suitability of the location. This assessment may include typical measurements such as pacing thresholds, sensing amplitudes, and tissue impedance. These functions may be accomplished with a delivery instrument in accordance with the embodiments shown in FIGS. 13 and 14. The working channel may also be used to assess the physiological suitability of the heart tissue. Temporary pacing of the heart 12 may also be performed at the location through the working channel.

After a determination that the location is suitable for lead implantation, an anchor mechanism 44 as is shown in FIG. 1 is advanced through the working channel with a delivery instrument 50 as is shown in FIGS. 2 - 6 in accordance with one embodiment of the present invention. A separate channel for fiber optic visualization may be used to assist the operator in guiding the delivery instrument 50 through the myocardial tissue 32. In one embodiment, the visualization channel is further used to avoid tissue structures, such as vessels, fat pads, nervous system tissue, and infarcts.

Optionally, the anchor mechanism 44 may be implanted without the aid of a delivery instrument as is described above, but rather with a curved suture needle. The proximal end of the tether 45 is attached to the needle, either directly or to a short length of suture attached to the needle. The needle is used to pierce the epicardium 28, is pushed through the myocardium 32 and drawn back through the epicardium 28, pulling the tether 45 through the myocardium 32. The tether 45 is cut from the needle and tensioned to bring the anchor mechanism 44 in contact with the epicardium 28. The lead 36 is threaded onto the tether 45 and advanced over the tether 45 as previously described.

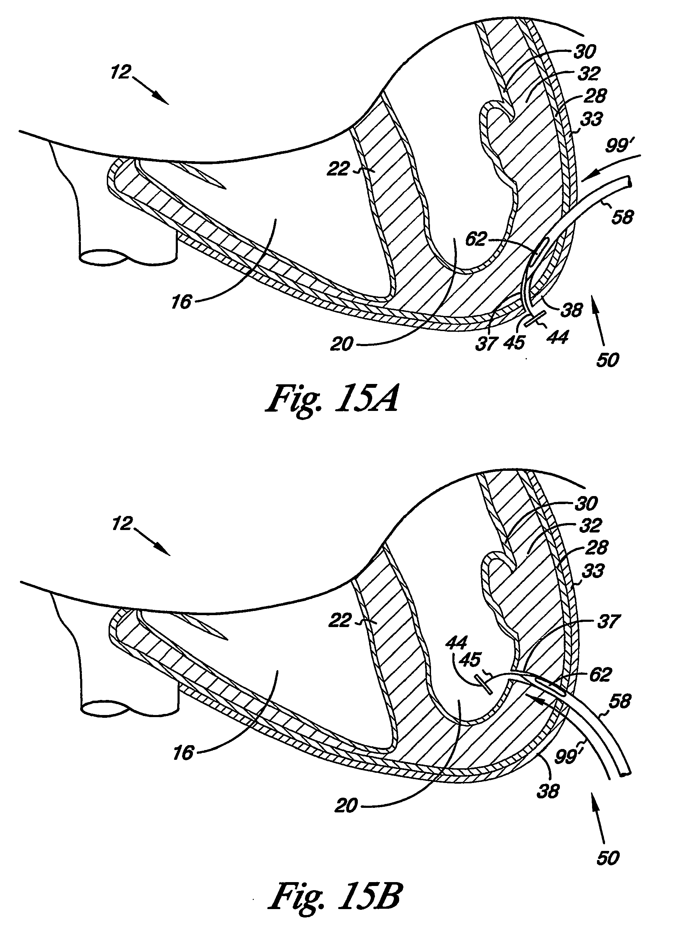

FIGS, 15A -15D illustrate the use of the delivery instrument 50. The needle portion 58 of the delivery instrument 50 is used to create a channel or tract 37 through the tissue of a patient's heart 12. FIG. 15A shows an epicardial-epicardial procedure, in which the needle portion 58 is inserted in the direction shown by the arrow 99', such that it both enters and exits the myocardium 32 through the epicardium 28. According to one embodiment, the needle tip 60 is rounded or blunt, as previously described to reduce trauma to the myocardial tissue 32 during advancement. During insertion through the myocardium 32, the anchor mechanism 44 is located within the nest 62. A tether 45 is attached to the anchor 44 and extends along the outside of the needle portion 58 and the handle 52.

FIG. 15B shows an epicardial-endocardial procedure, in which the needle portion 58 travels in the path indicated by the arrow 99', such that it enters the myocardium 32 through the epicardium 28 and exits through the endocardium 30. Again, the anchor mechanism 44 is located within the nest 62 and a tether 45 travels along the outside of the needle portion 58 and handle 52.

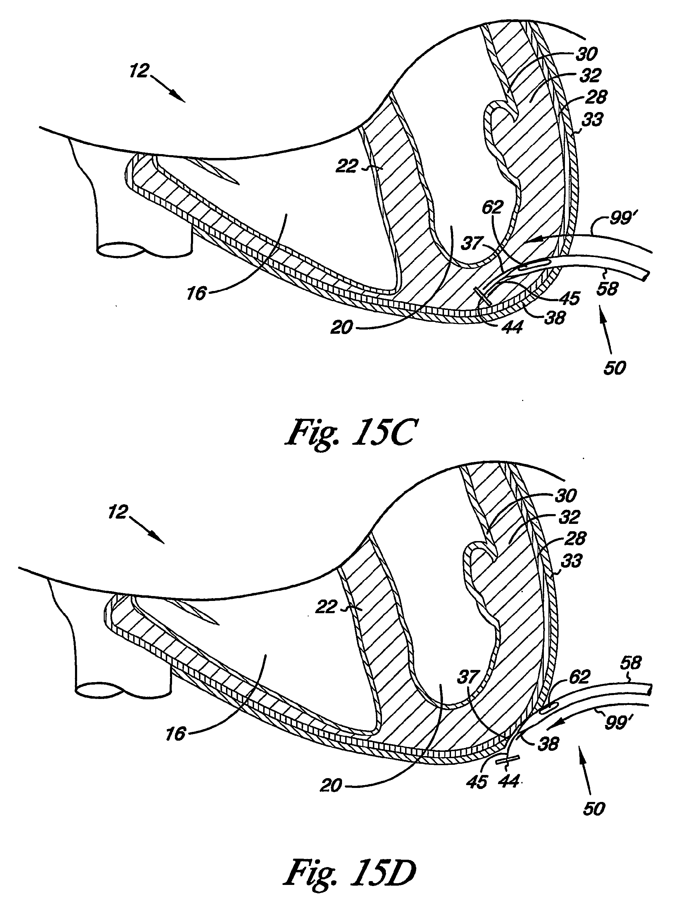

FIG. 15C shows an intra-myocardial procedure, in which the needle portion 58 travels in the path indicated by arrow 99', such that it enters the myocardium 32 through the epicardium 28. The anchor mechanism 44 is ejected into the myocardium 32 and the delivery instrument 50 is withdrawn. The anchor mechanism 44 remains positioned within the myocardium 32.

FIG. 15D shows a pericardial-pericardial procedure, in which the needle portion 58 travels in the path indicated by arrow 99' such that it enters and exits through the pericardium 33 without traversing the myocardium 32.

Once the delivery instrument 50 emerges through the epicardium 28 (or the endocardium 30, pericardium 33, or myocardium 32), the ejection mechanism 66 is activated to dislodge the anchor mechanism 44 from the nest 62. The delivery instrument 50 is then withdrawn back through the tract 37 and removed. The tether 45 is then tensioned to cause the anchor 44 to engage against the epicardium 28 (or endocardium 30, pericardium 33 or myocardium 32).

According to other embodiments (see FIGS. 7 - 10C, but referring generally to FIG. 7), a stylet or guide wire 106 in combination with a needle 102 and an anchor mechanism 108 is used to create the tract 37 through the myocardium 32 for delivery of the anchor mechanism 108. Once the distal end of the needle 104 and anchor mechanism 108 have emerged through the epicardium 28 (or endocardium 30, the myocardium 32 or pericardium 33), the stylet 106 is advanced. The ejector plug 116 engages the trailing edge 112 of the anchor mechanism 108 to eject the anchor mechanism 108 from the needle lumen 104. The needle 102 and stylet 106 are then withdrawn through the tract 37.

According to still other embodiments (see FIGS. 11 and 12, but referring generally to FIG. 11), a stylet or guide wire 182 in combination with an anchor mechanism 184 is used to create the tract 37 through the myocardium 32 for delivery of the anchor mechanism 184. Once the distal end 192 of the stylet or guide wire 182 and anchor mechanism 184 have emerged through the epicardium 28 (or endocardium 30, myocardium 32 or pericardium 33) the stylet 182 is withdrawn. The anchor mechanism 184 remains in place. The tether 196 is tensioned and the lead 36 advanced as described above. According to other embodiments, the stylet 182 and anchor mechanism 184 are advanced through the working channel rather than creating a separate tract 37.

Following delivery of the anchor mechanism 44 to the epicardial surface 28 (or the myocardium 32, the endocardial surface 30 or the pericardial surface 33) according to any of the aforementioned embodiments, the tether 45 is tensioned and the lead 36 advanced as described above. Following implantation of the myocardial lead 36, the tether 45 is tensioned and secured to the myocardial lead 36 to secure the myocardial lead 36 within the myocardium 32. According to other embodiments, the myocardial lead 36 is provided with additional means to secure the myocardial lead 36 within the myocardium 32 following delivery.

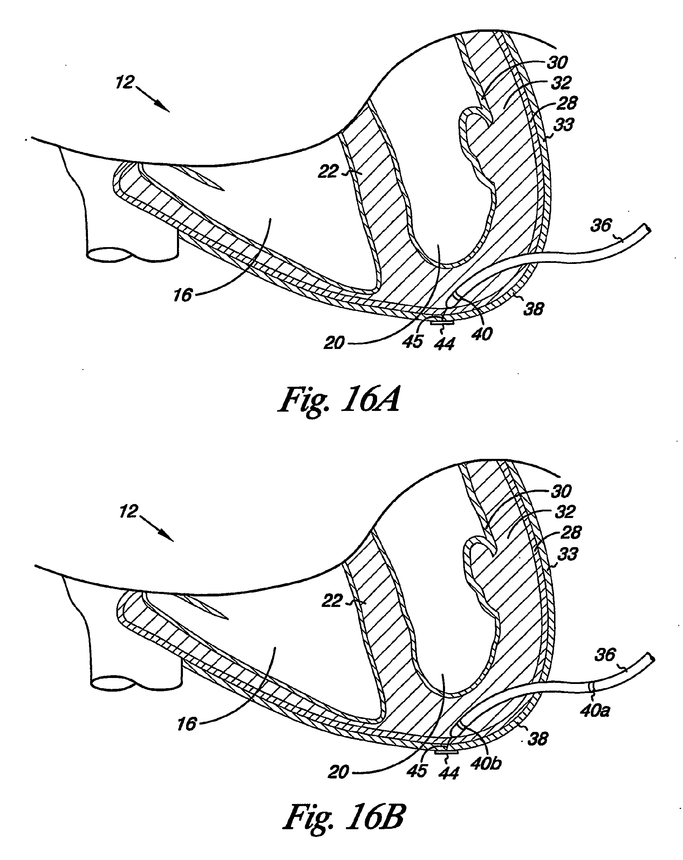

The lead 36 is positioned in the heart 12 and anchored, as described above, to position the lead electrode 40 in chosen locations in the heart 12. Although generally shown in implanted near the apex 38, the lead 36 may be implanted anywhere in the heart pacing therapy is needed. For example, the lead 36 may be implanted in the free wall of the left ventricle 20. According to one embodiment, shown in FIG. 16A, the lead 36 is configured as a unipolar lead. The electrode 40 is inserted into the heart tissue and is an active "can." The electrode 40 may be positioned in the myocardium 32, as shown, or may contact the epicardium 30 or the pericardium 33.

FIG. 16B shows another embodiment in which the lead 36 has two electrodes, a proximal anode 40a and a distal cathode 40b. According to one embodiment, the lead 36 is implanted in a pseudo-unipolar arrangement, such that the cathode 40b is positioned within the myocardial tissue 32 and the anode 40a is positioned on the lead body 36, but not in contact with the heart tissue.

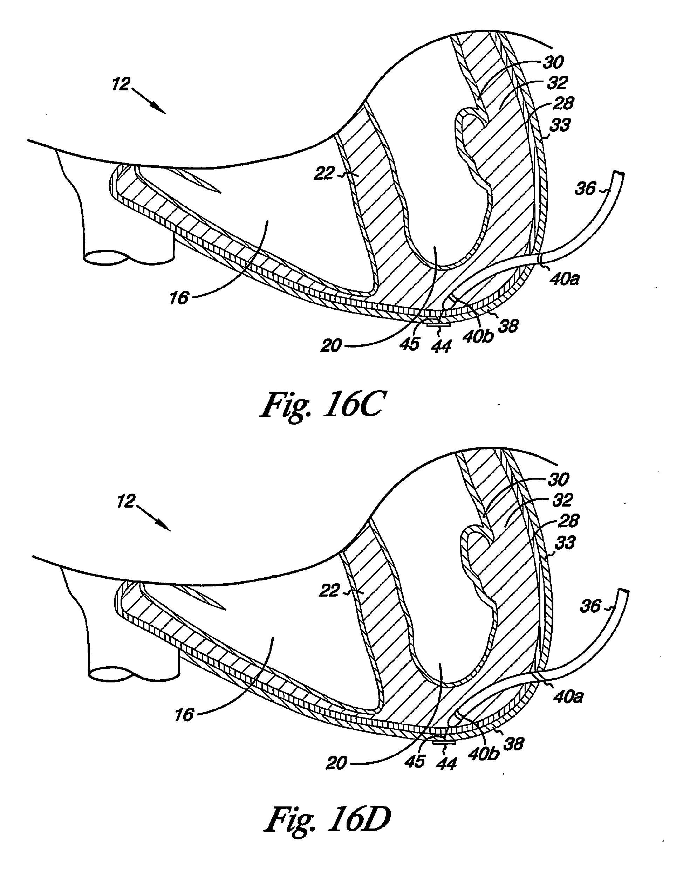

FIG. 16C shows another embodiment, in which the lead electrode arrangement is pseudo-unipolar, such that the cathode 40b is positioned in the heart and the anode 40a partially contacts the exterior epicardial tissue 28 of the heart 12.

FIG. 16D shows yet another pseudo-unipolar embodiment in which the cathode 40b is positioned in the myocardium 32 and the anode 40a is positioned in alignment with the outer layers of the epicardial tissue 28 of the heart 12. The anode 40a may, for example, straddle the epicardium 28 such that a portion anode 40a is within the heart tissue and another portion is exposed to a space outside of the heart 12.

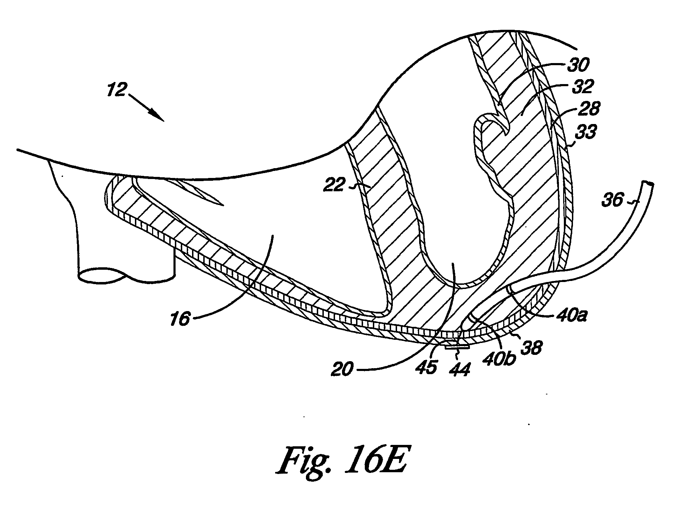

FIG. 16E shows another embodiment in which the lead 36 has a bipolar arrangement wherein the cathode 40b and anode 40a are completely within the myocardial tissue 32.

In one embodiment, the cathode 40b has an active electrode surface area of about 1 square mm. The cathode 40b is located near the distal tip 35 of the lead body 36. An electrode spacing between the cathode 40b and anode 40a of more than about 2 cm would generate a unipolar lead, as defined above. An electrode spacing of between about 1 and about 2 cm would generate a pseudo-unipolar lead, as defined above. An electrode spacing of less than about 1 cm would generate a bipolar lead, as defined above.

In the epicardium to epicardium configuration, the epicardium to endocardium configuration and the intramyocardial configuration, once implanted, the lead body 36 will serve to block the tract in the myocardium 32 created by the delivery instrument 50 and prevent further loss of blood from the heart 12. If it becomes necessary to reposition the lead 36 and create a second tract, a porous, clot-promoting "plug" material can be fed over the tether 45 into the tract 37 to serve as a seal. Following implantation of the myocardial lead 36, the tether 45 is tensioned and secured to the myocardial lead 36 to secure the myocardial lead 36 within the myocardium 32.

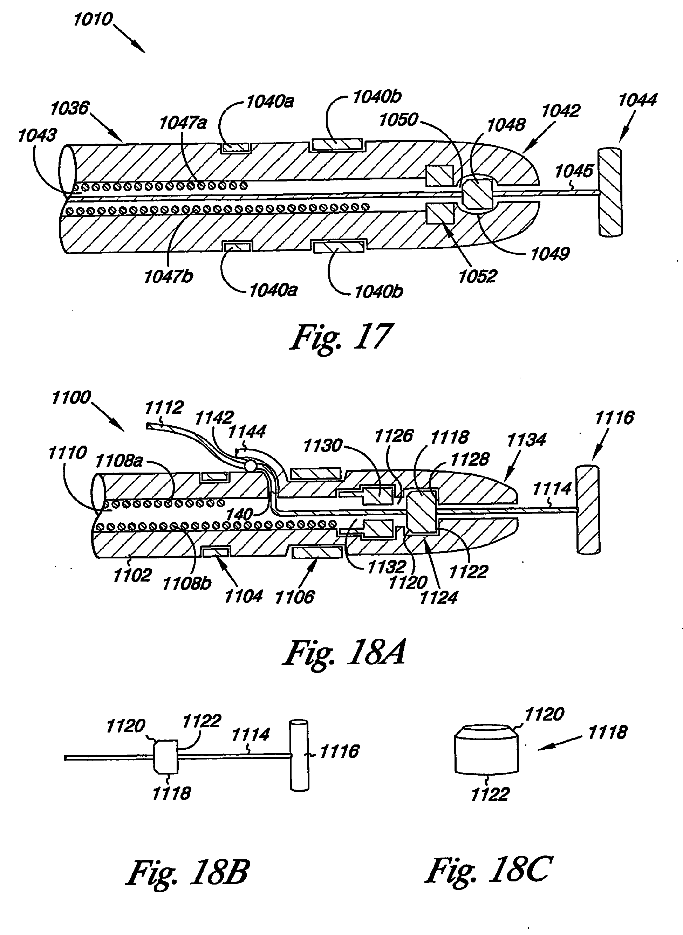

FIG. 17 shows a distal portion of a myocardial lead attachment system 1010 according to another embodiment of the present invention. A lead 1036 includes a pair of electrodes, a proximal anode 1040a and a distal cathode 1040b. A pair of coiled conductive members 1047a and 1047b extends longitudinally through the lead 1036 and are electrically coupled to the anode 1040a and the cathode 1040b, respectively. The lead 1036 has a lumen 1043 extending longitudinally therethrough for receiving the tether 1045. The tether 1045 is provided with a distal lock for restraining motion of the lead 1036 with respect to the tether 1045 in either of a proximal or a distal direction. The distal lock include a lock structure 1048 formed on the tether. A distal end 1042 of the lead 1036 is provided with a lock housing 1049 adapted to receive the lock 1048. As the lead 1036 is advanced over the tether 1045, the distal tip 1042 of the lead 1036 approaches the lock 1048. The distal tip 1042 is resiliently deflectable such that the lock 1048 is received in the lumen 1043. The distal end 1042 of the lead 1036 advances slightly beyond the lock 1048 until the lock 1048 engages the lock housing 1049. The lock 1048 and lock housing 1049 mate and form a locking arrangement that retains the lead 1036 in a stable position in the myocardium 32. The locking arrangement is desirable because it helps to reduce migrations of the distal tip 1042 of the lead body 1036 due to contractions of the heart 12 and patient movement.

The lock arrangement can be any configuration that securely attaches the lead 1036 to the tether 1045. In the present embodiment, the lock housing 1049 is a cavity 1050 formed in the internal lumen 1043 proximal to the distal tip 1042 of the lead 1036. A non-resilient internal ring 1052 is positioned about the lumen 1043 proximal to the cavity 1050. The ring 1052 has a diameter smaller than the size of the lock 1048. While the distal tip 1042 of the lead 1036 is sufficiently resilient to expand and allow the lock 1048 to enter the lead 1036, the ring 1052 does not deflect, and the lead 1036 is prevented from advancing past the lock 1048.

FIGS. 18A - 18C show a distal portion of a myocardial lead attachment system 1100 in accordance with another embodiment of the present invention. Myocardial lead attachment system 1100 includes many of the features of the embodiment shown in FIG. 17, including a lead body 1102 having a proximal anode 1104 and a distal cathode 1106, a pair of coiled conductive members 1108a and 1108b. The coiled conductive members 1108a and 1108b are coupled to the proximal anode 1104 and distal cathode 1106, respective. The lead body 1102 further includes an internal lumen 1110 for receiving a tether 1112. A distal end 1114 of the tether 1112 is coupled to an anchor mechanism 1116. Shown more clearly in FIGS. 18B and 18C, a lock 1118 is formed on the tether 1112 near the distal end 1114 and is shaped as a three-dimensional wedge. A forward face 1120 of the lock 1118 is ramped and pointed toward a proximal end of the tether 1112 (not shown), while a trailing face 1122 of the wedge-shaped lock 1118 is generally perpendicular to the tether 1112.

Returning to FIG. 18A, the lead 1102 is provided with a cavity 1124 forming a lock housing 1126 in the lumen 1110. The cavity 1124 has a distal side 1128 perpendicular to the length of the lead 1102. A non-resilient ring 1130 is positioned at a proximal side 1132 of the lock housing 1126. The lead 1102 includes a through hole 1140 extending from the lumen 1110 through the lead body 1102 proximal to the lock housing 1126, near the location where the lead 1102 exits the epicardium 28, and a flap or pocket 1144 adjacent the through hole 1140.

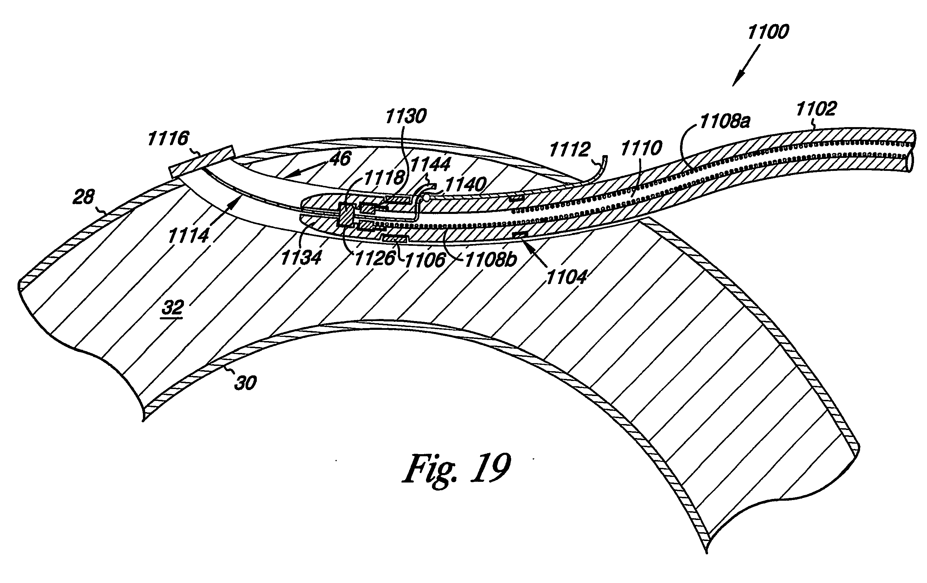

FIG. 19 shows a distal portion of a myocardial lead attachment system 1100 implanted in the myocardium 32. Prior to lead implantation, the tether 1112 is drawn out of the through hole 1140. The lead body 1102, now threaded onto the tether 1112, is then advanced over the tether 1112 through a tract 46 in the myocardium 32. As a distal tip 1134 of the lead 1102 advances to the wedge-shaped lock 1118, the ramped side 1120 of the wedge-shaped lock 1118 deforms the distal tip 1134 of the lead 1102, allowing the lock 1118 to enter and pass through the lumen 1110 while the lead 1102 continues to advance forward. The lock 1118 encounters and mates with the lock housing 1126, as the ring 1130 prevents further distal advancement of the lead 1102 beyond the lock 1118. The tip 1134 returns to its original shape due to its resilient nature. When the perpendicular side 1128 of the lock housing 1126 engages the lock perpendicular trailing face 1122, further longitudinal sliding of the lead 1102 in a proximal direction is prevented. Furthermore, lock 1118, when retained in lock housing 1126, substantially blocks the passage of any fluids into the lead lumen 1110.

When the lead 1102 has been properly positioned against the lock 1118 such that the lock 1118 is mated with in the lock housing 1126, the tether 1112 is placed under tension relative to the lead 1102 by applying a tensile force to the portion of the tether 1112 exiting through the through hole 1140. The tensioned tether 1112 is then fixed to the lead 1102 at an attachment site near the through hole 1140. In this manner, the myocardial lead 1102 is fixed along the tether 1112 between the lock 1118 and the attachment site, which retains the lead 1102 in a consistent position within the myocardium 32.

According to one embodiment, the tether 1112 is fixed to the lead 1102 by forming a knot 1142 in the tensioned tether 1112 just outside the through hole 1140 (See FIG. 18A). The knot 1142 is of a sufficient size to be retained outside of the through hole 1140 even while under tension. The tensile force draws the knot 1142 against the lead 1102 to a stable, fixed position. Again, this configuration secures the lead 1102 between the lock 1118 and the knot 1142 at the through hole 1140. The knot 1142 resides within the flap 1144. This prevents irritation to the myocardial tract 1046 that may be caused by movement or sliding of the knot 1142 within the tract 1046.

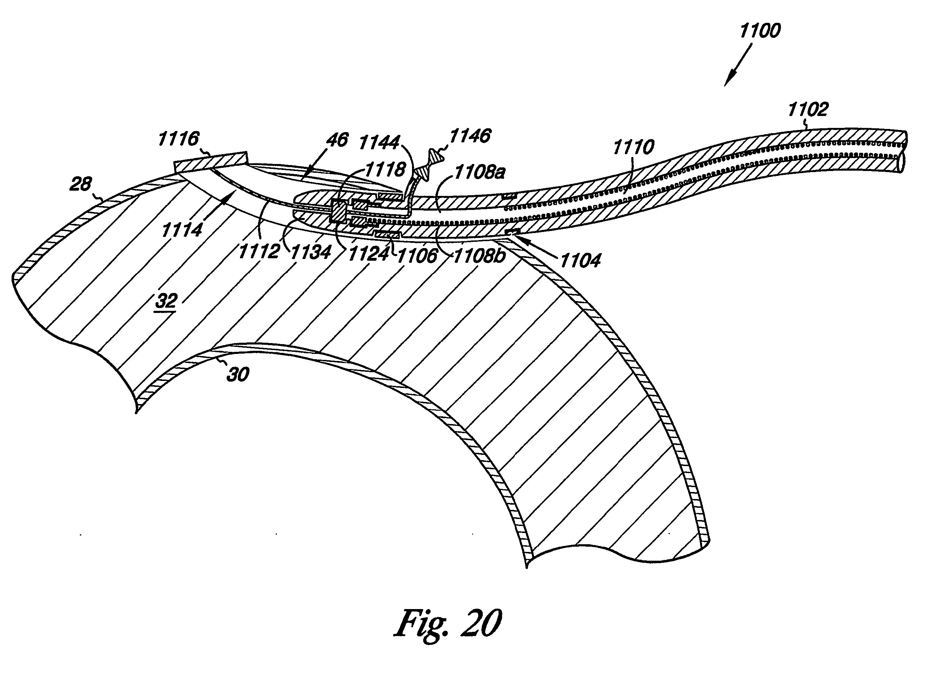

FIG. 20 shows another embodiment in which a slidable cinch 1146 is positioned at the exit hole 1140. The slidable cinch 1146 is permanently attached, for example, by crimping, to the tether 1112 to provide the desired tension on the lock 1118 to hold the lead 1102 in place. In all of these embodiments, once the fixation tension has been placed on the tether 1112, the remaining tether 1112 material can be cut and removed.

The lock structure 1118 may take various shapes, and may be a separate member from the tether 1112, for example a clip or other structure, fastened to the tether 1112. In still other embodiments, the lock 1118 and lock housing 1126 may have other complementary mating shapes, which operate to inhibit motion of the lock 1118 with respect to the lock housing 1126.

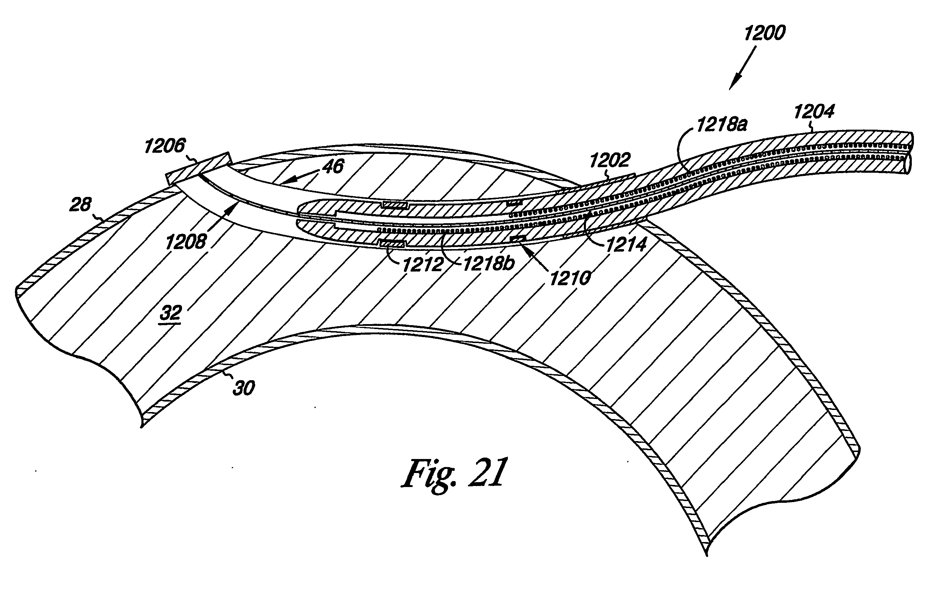

FIG. 21 shows a distal portion of a myocardial lead attachment system 1200 according to another embodiment of the present invention, including a distal lead fixation sleeve 1202. The lead attachment system 1200 includes a lead body 1204, an anchor mechanism 1206 and a tether 1208 as previously described. The lead body 1204 includes a proximal anode 1210, a distal cathode 1212 and a pair of coiled conductive members 1218a and 1218b as previously described, as well as an internal lumen 1214 for receiving the tether 1208. The anchor mechanism 1206 and tether 1208 are advanced through a tract 1046 in the myocardium 32 as previously described. The lead body 1204 is advanced over the tether 1208. The fixation sleeve 1202 is passed distally down the length of the lead 1204 to the vicinity of the epicardium 28 exit site and partially inserted through the epicardium 28. The sleeve 1202 is sutured into the epicardial tissue 28 to form a lock arrangement to restrain motion of the lead 1204 with respect to the tether 1208 in either of a proximal or a distal direction. The sleeve 1202 may be stitched to the epicardial tissue 28 in such a way as to bring about sufficient compression on the lead body 1204 to form a firm attachment at the exit site. The anchor 1206 and tether 1208 may be removed or may simply be left in place.

The preceding embodiments generally describe fixation systems for fixing the distal end of the lead in position. According to other embodiments of the present invention, proximal fixation systems are provided for fixing the proximal end of the lead in a selected position.

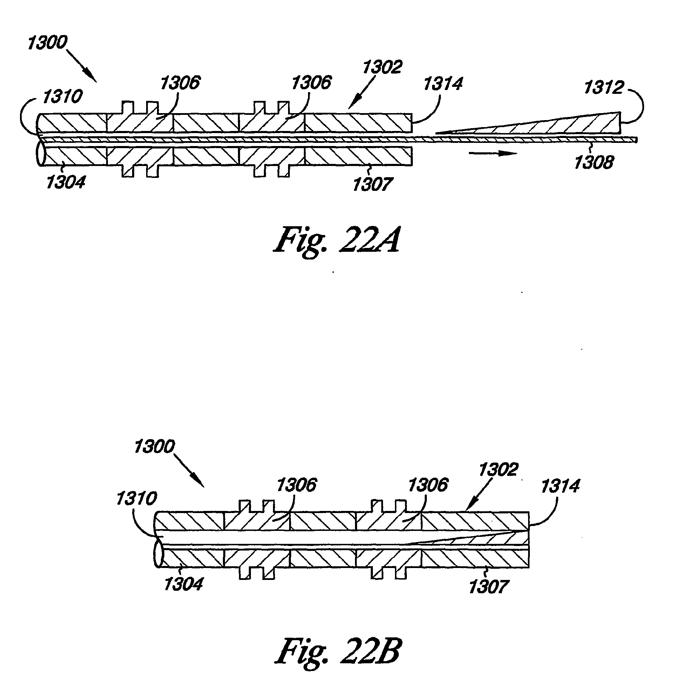

FIGS. 22A-22B show a myocardial lead fixation system 1300 according to another embodiment of the present invention. A proximal end 1302 of a myocardial lead 1304 is provided with a plurality of terminal connectors 1306 for forming an electrical connection with a pacing device (not shown), and a proximal-most terminal pin 1307. A tether 1308 is coupled at a distal end to an anchor mechanism (not shown) and extends proximally through a lumen 1310 in the lead 1302 and terminal pin 1307. The system 1300 includes a lock structure 1312 shaped like a wedge. After the lead 1304 has been advanced over the tether 1308 into the myocardial tract 1046, the tether 1308 is placed under tension relative to the lead 1304. The lock structure 1312 is inserted into the lumen 1310 at the terminal pin 1307, fixing the tether 1308 in frictional engagement against an internal wall of the lumen 1310 and forming a proximal locking arrangement to restrain motion of the lead 1302 with respect to the tether 1308 in either of a proximal or a distal direction. Any portion of the lock structure 1312 may be cut along with the trailing length of the tether 1308 flush against a proximal end 1314 of the terminal pin 1307. The terminal pin 1307 and terminal connectors 1306 may be inserted into the pulse generator 34 without interference from the proximal locking arrangement (See FIG. 1).

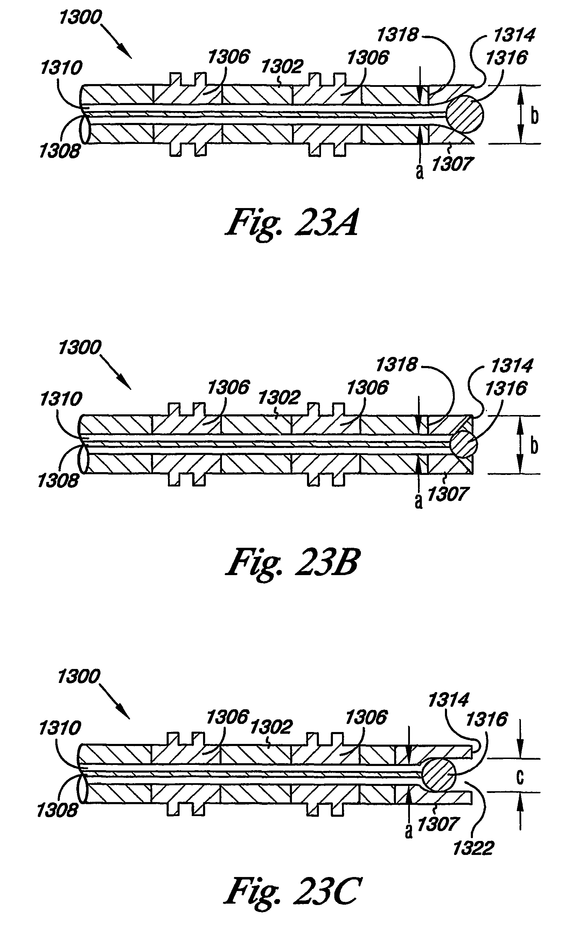

FIGS. 23A - 23C show additional embodiments of proximal locking arrangements in which in place of the wedge-shaped lock structure 1312, a knot 1316 or other mechanical stop is formed on the tether 1312 of a size sufficient to be retained in frictional engagement with the lead 1302 outside of the lumen 1310. The tether 1308 is tensioned and the knot 1316 is formed to restrain motion of the lead body 1302 with respect to the tether 1308 in either of a proximal or a distal direction. As with respect to the embodiment shown in FIG. 22A and 23B, it is important that the proximal locking arrangement not interfere with insertion of the terminal pin 1307 into the pulse generator 34.

According to one embodiment, as shown in FIG. 23A, the lumen 1310 tapers outwardly proximally to form a housing to receive the knot 1316. At a mid region 1318 of the terminal pin 1307, approximately .024 inches distal to a proximal tip 1314 of the terminal pin 1307, the wall of the terminal pin 1307 is about .029 inches thick and the lumen 1310 has an internal diameter a of about .022 inches. The lumen 1310 tapers outwardly such that at the proximal tip 1314 the lumen 1310 has an internal diameter b of about 0.050 inches and the wall of the terminal pin 1307 is about .010 inches thick. According to one embodiment, a single knot 1316 of the tether 1308 is able to be concealed within the lumen 1310 but is yet of a size to be prevented from sliding longitudinally within the lumen 1310 beyond the mid region 1318.

FIG. 23B shows another embodiment, in which the lumen 1310 angles outwardly at about 45° from the mid region 1318 to the proximal tip 1314. The internal diameter a of the lumen 1310 at the mid region 1318 is about .024 inches. The internal diameter b of the lumen 1310 at the proximal tip 1314 is about .045 inches. According to one embodiment, a single knot 1316 of the tether 1308 protrudes approximately .020 inches from the distal end 1320 of the terminal pin 1307. According to yet another embodiment, the internal diameter a of the lumen 1310 at the mid region 1318 is about .0197 inches and the internal diameter b of the lumen 1310 at the proximal tip 1314 is about .0315 inches.

FIG. 23C shows another embodiment, in which the lumen 1310 is chamfered at an angle of about 45° distal to the proximal tip 1314. The lumen 1310 angles outwardly, forming a housing 1322 having an internal diameter c greater than the internal diameter of the lumen 1310. According to one embodiment, the internal diameter a of the lumen 1310 is about .0210 inches and the lumen 1310 angles outwardly at about 45° for about .012 inches. The internal diameter c of the housing 1322 is about .045 inches, and extends about .040 inches to the distal tip 1320. According to one embodiment, a single knot 1316 of the tether 1308 is able to be concealed within the housing 1322.

According to one embodiment, the tether 1308 is formed of 0.0095 inch diameter polypropylene and a single knot 1316 of the tether 1308 is about .040 inches long by about .030 inches wide.

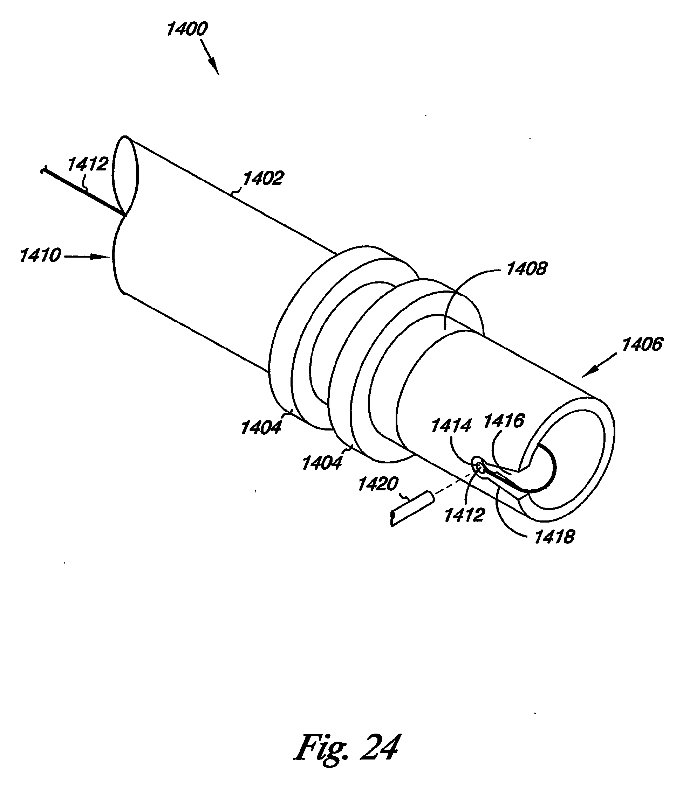

FIG. 24 is a perspective view of a proximal locking arrangement at a proximal portion of a myocardial lead attachment system 1400, according to one embodiment of the present invention. The system 1400 includes a lead body 1402 having a terminal connector 1404 and a terminal pin 1406 positioned at a proximal end 1408 of the lead 1402. A lumen 1410 extends through the lead body 1402 and terminal pin 1406 for receiving a tether 1412. The terminal pin 1406 of the lead 1402 includes a tether lock pin 1414 at a proximal end 1408 of the lead 1402. The lock pin 1414 includes a first channel 1416 through which the tether 1412 passes. The lock pin 1414 also includes a side groove 1418 into which the tether 1412 is placed under slight tension. Once so positioned, an insert pin 1420 is inserted into the lock pin 1414 and firmly pressed forward. The insert pin 1420 fixes the tether 1412 at the lock pin 1414. In one embodiment, the insert pin 1420 also operates to cut the excess length of the tether 1412.

According to one embodiment, both a distal and a proximal attachment system are used to secure the lead to the tether. The combination of a proximal locking arrangement and a distal locking arrangement stabilize the lead within the heart 12 and reduce the likelihood of migrations of the lead from the implant site. Furthermore, the lead can be positioned in the heart 12 in a variety of configurations, including epicardial-epicardial, as is shown in the preceding figures, or epicardial-endocardial, intra-myocardial and pericardial-pericardial, as is described above.

Referring generally to FIG. 1 the anchor mechanism 44 is made from any biocompatible material known in the art suitable for chronic implantation. The tether 45 is formed from any biocompatible material known in the art having, a strength and flexibility sufficient to guide and secure the lead 36 within the myocardium 32. In one embodiment, the tether 45 is formed from any conventional suture material known in the art.

Over time, collagenous encapsulation tissue ("scar tissue") forms around the system 10. The formation of such scar tissue sometimes acts to secure the lead 36 in position.

According to one embodiment, the anchor mechanism 44 is made from a material formulated to dissolve or be absorbed over a period of time greater than a period of time necessary for the formation of scar tissue around the myocardial lead 36 following implantation. In one embodiment, the material is configured to dissolve in a period of time greater than a period of time necessary to secure the myocardial lead 36 to the myocardium 32 by scar tissue formation around the myocardial lead 36. Such material may be any bioabsorbable or biodegradable material, including, for example, polyglycolide ("PGA"), polylactide ("PLA"), polydioxanone ("PDA"), or polylactide-co-glycolide. In one embodiment, any combination of these polymers is used.

Dissolution or bioabsorption of the anchor mechanism 44 releases the tether 45 and lead 36. Although doing so reduces the level of fixation of the lead 36 within the myocardium 32, the lead 36 remains substantially secured within the myocardium 32 by epicardial and/or myocardial scar tissue. The lead 36 no longer need be detached from the anchor mechanism 44 prior to removal, as would otherwise be necessary. Rather, the lead 36 need only be separated from the surrounding scar tissue. Accordingly, this will facilitate later removal of the lead 36, if necessary.

In another embodiment, the material of the anchor mechanism 44 includes an agent, biologic material or drug, released as the anchor mechanism 44 dissolves, which would alter the local environment of the lead implantation site. This material could be selected to include anti-inflammatory material, angiogenic factors or cellular growth factors or modifiers to enhance healing and low stimulation thresholds.

In one embodiment, the tether 45 is made from any bioabsorbable or biodegradable material, such that a portion of the tether 45 located outside of the lead 36 dissolves or is absorbed over time. Such materials include, for example, PGA, PLA, PDA, or polylactide-co-glycolide as previously described. Dissolution of the tether 45 releases the lead 36 from the anchor mechanism 44. Again, the lead 36 then need only be separated from surrounding scar tissue prior to removal. The anchor mechanism 44 may be removed as well, or may remain in place, encapsulated by scar tissue. In one embodiment, both the anchor mechanism 2044 and the tether 2045 are made from a dissolvable or absorbable material.

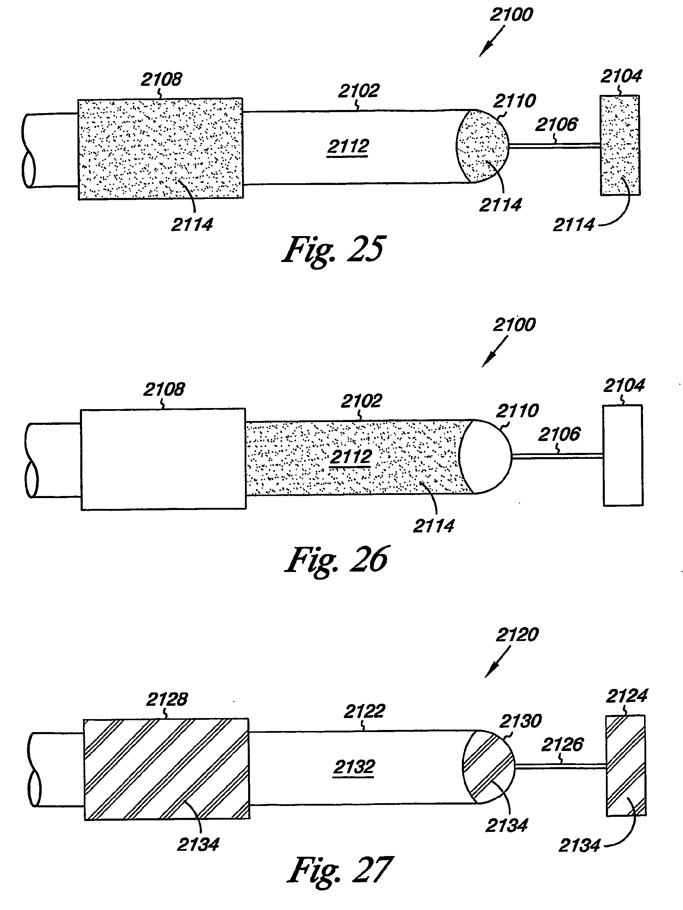

FIG. 25 shows a side view of a distal portion of a myocardial lead attachment system 2100 in accordance with another embodiment of the present invention. Attachment system 2100 includes many of the same features as the system 10 shown in FIG. 1, including a lead 2102, an anchor mechanism 2104 and a tether 2106 for securing the lead 2102 within the myocardium 32. The lead 2102 further includes a proximal electrode 2108, a distal electrode 2110 and an outer insulating sheath 2112.

The system 2100 is further provided with a porous or roughened surface feature(s) 2114 into which collagenous encapsulation tissue ("scar tissue") invades, resulting in natural tissue anchoring. The scar tissue encapsulation that forms about the roughened surface feature(s) 2114 provides a gripping action strengthened as the encapsulation tissue invades the surface feature(s) 2114. Natural tissue anchoring strengthens the fixation between the lead 2036 and the heart 12, reducing dislodgment and repositioning of the lead 2102.

In the embodiment shown in FIG. 25, the proximal electrode 2108, the distal electrode 2110 and anchor mechanism 2104 are provided with surface feature 2114. According to other embodiments, portions of either or both of the proximal electrode 2108 and distal electrode 2110 in contact with heart tissue are provided with surface feature 2114.

Putting surface feature 2114 on the anchor mechanism 2104 increases fixation of the anchor mechanism 2104 to the heart 12 and reduces the likelihood of re-entry of the anchor mechanism 2104 into the tract. According to other embodiments, either or both of the anchor mechanism 2104 and the lead 2102 include surface feature 2114. In those embodiments in which the anchor mechanism 2104 and/or tether 2106 are dissolvable, as described above, the natural tissue anchoring improves lead fixation after either or both of the anchor mechanism 2104 and tether 2106 have dissolved.

FIG. 26 shows another embodiment in which the outer insulating sheath 2112 is provided with surface feature 2114. Any portion of the lead body 2102 that passes through the epicardium 30 contains the surface feature 2114. According to another embodiment, surface feature 2114 is provided over the entire surface of the lead body 2102 extending from the pulse generator 34 to the epicardium 30 (see FIG. 1). Where the insulating sheath 2112 is provided with surface feature 2114, open channels that would provide electrical communication between internal conductor wires coupled to the proximal electrode 2108 and distal electrode 2110 (not visible in FIG. 2) and the myocardium 32 should be avoided.

Lead/electrode substrates forming a porous or roughened surface can be provided in a number of ways to form surface feature 2114. In one example, the distal electrode 2110, proximal electrode 2108, sheath 2112 or anchor mechanism 2104 can be sand/grit blasted to bring about the surface feature 2114. The rough or textured character of the surface feature 2114 encourages tissue ingrowth. According to other embodiments, (not shown) circumferential grooves or other discontinuities form surface feature 2114.

According to another embodiment, the distal electrode 2110, proximal electrode 2108 or anchor mechanism 2104 can be fabricated from fused metallic particles so as to provide internal voids and channels forming surface feature 2114 and into which tissue ingrowth takes place. According to another embodiment, the electrodes 2108 and 2110 or anchor mechanism 2104 can be fabricated from metallic wire and/or screen mesh components that when compressed into an "electrode shape" create internal voids and channels to form surface feature 2114.

Once tissue ingrowth has occurred, unwanted motion of the lead 2102 relative to the heart 12 will be reduced or eliminated. In addition to reducing unwanted relative motion, a natural tissue anchoring feature such as that formed about surface feature 2114 acts as a back-up anchor should the tether 2106 primarily holding the lead body 2102 in place stretch or break. Where either or both of the anchor mechanism 2104 or tether 2106 are intended to dissolve or be absorbed over time as discussed above, a natural tissue anchoring feature formed on the lead 2102 improves lead 2102 stability following dissolution or bioabsorption.

FIG. 27 shows a side view of a distal portion of a myocardial lead attachment system 2120 in accordance with another embodiment of the present invention. Attachment system 2120 is generally similar to attachment system 2100 shown in FIGS. 25 and 26 and includes a lead 2122, an anchor mechanism 2124 and a tether 2126. The lead body 2122 includes a proximal electrode 2128, a distal electrode 2130 and an outer insulating sheath 2132. The proximal electrode 2128 and distal electrode 2130 are provided with a biocompatible conductive coating 2134. The coating 2134 may be formed with a smooth surface, as is shown in FIG. 27, or may be formed with a rough or porous surface as is previously described. The conductive coating 2134 encourages growth and formation of the scar tissue, stabilizing the lead 2120 within the myocardium 32.

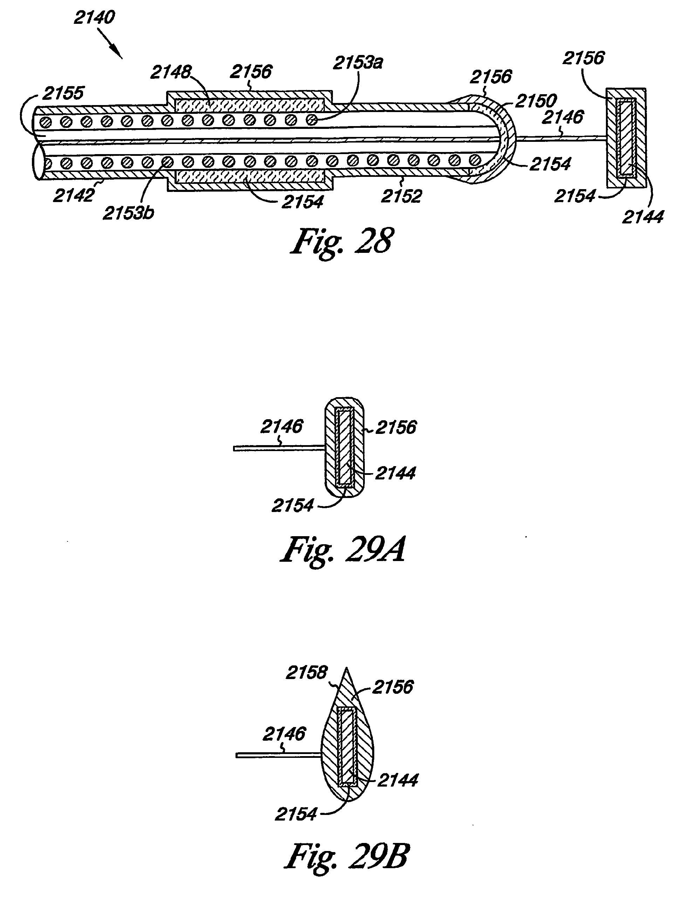

FIG. 28 shows a distal portion of a myocardial lead attachment system 2140 according to another embodiment of the present invention. The attachment system 2140 is generally similar to that shown in FIG. 2 and includes a lead 2142, an anchor mechanism 2144 and a tether 2146. The lead 2142 includes a proximal electrode 2148, a distal electrode 2150 and an outer insulating sheath 2152. Also shown are a pair of coiled conductive members 2153a and 2153b which are electrically coupled to the electrodes, 2148 and 2150, respectively. Also shown is a central lumen 2155 extending through the lead 2142 for receiving the tether 2146.

The lead 2142 is provided with a roughened surface feature 2154 formed on the proximal electrode 2148, distal electrode 2150 and/or anchor mechanism 2144. A rapidly dissolvable outer coating 2156 is formed over the surface feature 2154. Such a rapidly dissolvable coating 2156 may be formed of a material that is water soluble. The rapidly dissolvable coating 2156 provides a smooth outer surface, masking any surface features, including roughened surface feature 2154, to facilitate passage of the lead 2142 and anchor mechanism 2144 through the myocardium 32 during implantation. Following implantation, the coating 2156 rapidly dissolves, revealing the surface features 2154 to permit tissue ingrowth at the surface feature 2154. According to other embodiments, the coating 2156 is formed on the lead body 2102 and/or anchor mechanism 2144, or anywhere the surface feature 2154 is formed.

FIG. 29A shows another embodiment in which the rapidly dissolvable coating 2156 has a first implant friendly shape or outer profile. Not only does the dissolvable coating 2156 mask the roughened surface feature 2154, as described above, the coating 2156 forms a profile about the anchor mechanism 2144 to facilitate implantation and passage through the myocardium 32. The coating 2156 is smooth and has rounded edges chosen to reduce trauma to the myocardium 32 as the anchor mechanism 2144 passes through the myocardial tissue 32 during insertion. Once the anchor mechanism 2144 is in place, the coating 2156 dissolves to expose the porous or roughened surface feature 2154. According to other embodiments, the anchor mechanism 2144 does not include a roughened surface feature 2154, or is dissolvable as previously described.

FIG. 29B shows another embodiment in which the coating 2156 is applied to the anchor mechanism 2144 to generate a shape configured to facilitate tissue dissection. In the embodiment shown, coating 2156 is shaped to form a sharp edge 2158. An anchor mechanism 2144 including this feature may be used in conjunction with a stylet delivery instrument as described above to form a tract through the myocardium 32. Upon implantation, the coating 2156 dissolves to expose the anchor mechanism 2144.

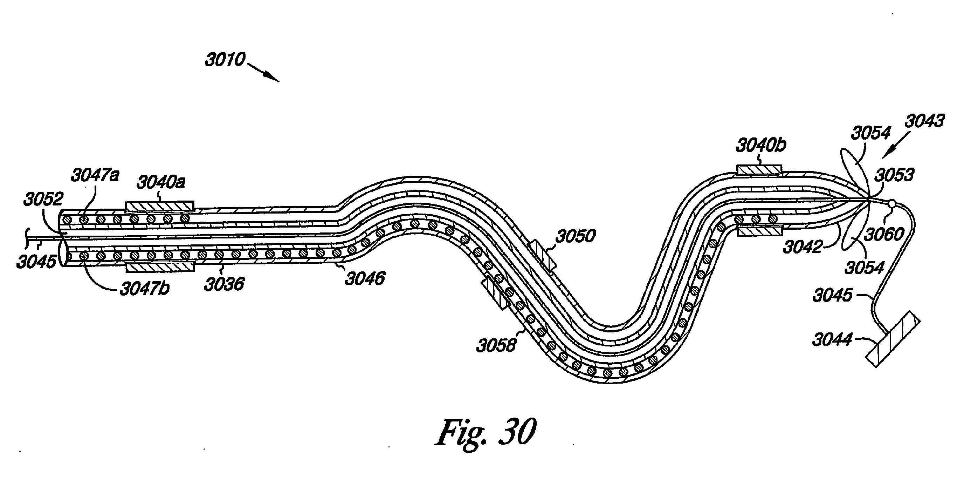

FIG. 30 shows a sectional view of a distal portion of a myocardial lead attachment system 3010. The lead 3036 includes one or more proximal electrodes 3040a positioned proximal to one or more distal electrodes 3040b located near a distal tip 3042 of the lead 3036. A pair of coiled conductive members 3047a and 3047b are coupled to the proximal electrode 3040a and the distal electrode 3040b, respectively, and are protected by an outer insulating sheath 3046. The lead 3036 further includes one or more drug collars 3050 for releasing therapeutic agents following implantation. According to other embodiments, the lead 3102 does not include the drug collar 3050. The lead 3036 also includes a lumen 3052 extending therethrough for receiving a tether coupled to an anchor mechanism.

A fixation mechanism 3043 is positioned at the distal tip 3042 of the lead 3036. The fixation mechanism 3043 includes a through-hole 3053 to receive the tether 3045, which also extends through the lead lumen 3052. The fixation mechanism 3043 is formed of a plurality of tines or fins 3054 extending outwardly from the lead 3036. In a first configuration (not shown), the tines 3054 are collapsed against the lead 3036 to facilitate advancement of the lead 3036 through the heart 12. In a second configuration, illustrated in FIG. 30, the tines 3054 extend radially outwardly from the lead 3036 and resist movement of the lead 3036 through the heart 12. Generally, the fixation mechanism 3043 has a first diameter in the first configuration and a second diameter in the second configuration greater than the first diameter. According to other embodiments (not shown), the fixation mechanism 3043 is positioned on the lead 3036 proximal to the distal tip 3042.

In one embodiment, a distally directed axial force drawing or pushing the lead 3036 forward or in a distal direction through the myocardium 32 retains the fixation mechanism 3043 in the first configuration. Likewise, a proximally directed force pulling the lead 3036 back or in a proximal direction deploys the fixation mechanism 3043 in the second configuration. In another embodiment, movement through the myocardium 32 in a distal direction collapses the fixation mechanism 3043 while movement through the myocardium 32 in a proximal direction frictionally engages the fixation mechanism 3043, deploying it to the second configuration. In other embodiments, rotating the fixation mechanism 3043 in a first direction retains the fixation mechanism 3043 in the first configuration and rotating or pulling the fixation mechanism 3043 in a second direction deploys the fixation mechanism 3043 into the second configuration.

The lead 3036 includes a bias region 3058 proximal to the fixation mechanism 3043. Bias region 3058 is a two or three-dimensional feature formed by a curvature of the lead 3036. The bias region 3058 absorbs axial loading at a proximal end 3035 of the lead 3036 without translating it into motion at the distal tip 3042. This reduces intra-myocardial electrode motion and strain on the fixation mechanism 3043 and distal migration of the lead 3036. The proximal electrode 3040a is positioned proximal to the bias region 3058 and the distal electrode 3040b is positioned distal to the bias region 3058. In other embodiments, the electrodes 3040a and 3040b are both positioned proximal or distal to the bias region 3058. In another embodiment (not shown), the lead 3036 does not include the bias region 3058.

A lock 3060 is formed on the tether 3045 and is receivable in a lock housing formed on the lead 3036 to produce a locking arrangement (not shown). Suitable lock arrangements are described above. The through-hole 3053 in the fixation mechanism 3043 is sized to receive and pass over the lock 3060.

According to one embodiment, either or both of the anchor mechanism 3044 and tether 3045 are dissolvable. Suitable dissolvable anchor mechanisms and tethers are described above. Following dissolution of either or both of the anchor mechanism 3044 and the tether 3045, the fixation mechanism 3043 provides stability to the implanted lead 3102.

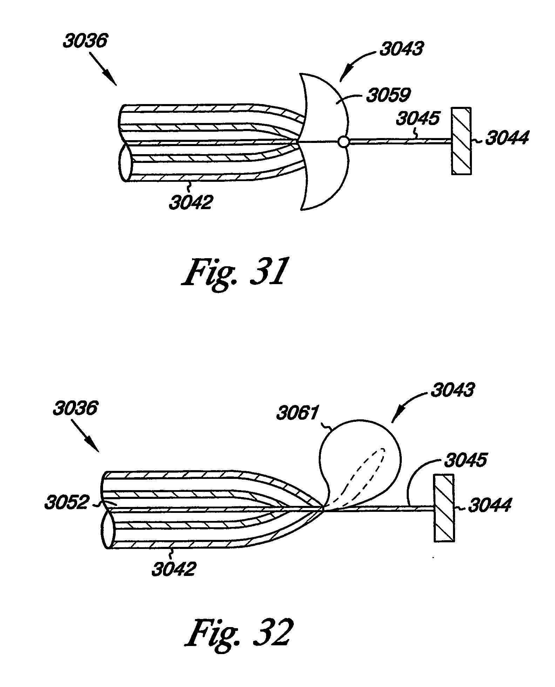

FIG. 31 shows another embodiment of the fixation mechanism 3043, in which the fixation mechanism 3043 is shaped as a ribbed tent or umbrella 3059. In the first configuration (not shown), the umbrella 3059 is deflated and collapsed against the lead 3036. In the second configuration, illustrated in FIG. 3, the umbrella 3059 is inflated and expanded radially outward. In still other embodiments (not shown), the fixation mechanism 3043 is shaped like a disc or bar or other shape chosen to advance easily through the heart 12 in a first configuration and engage the heart 12 in a second configuration to the secure the lead 3036 in place.

FIG. 32 shows another embodiment of the fixation mechanism 3043, in which the fixation mechanism 3043 is an inflatable balloon 3061. In the first configuration, shown in dotted lines, the balloon 3061 is deflated. In the second configuration, the balloon 3061 is inflated so as to have a greater diameter than in the first configuration. The balloon 3061 may be inflatable via fluids, including air, water or other fluids or gases. Such balloon deployment fluids are carried to the balloon 3061 through the lead lumen 3052. While shown superimposed over the tether 3045, the balloon 3061 would reside to the side of the tether 3045.

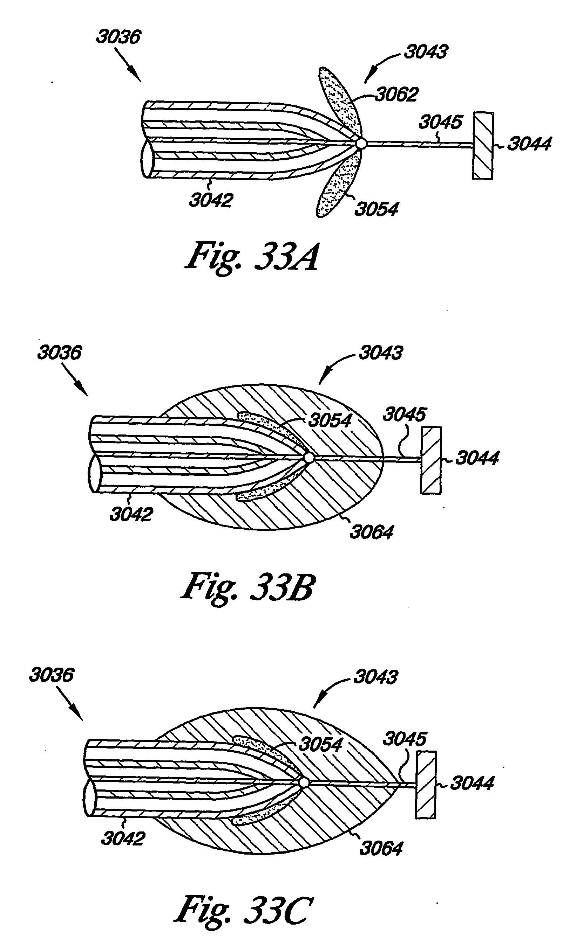

FIG. 33A shows another embodiment of the fixation mechanism 3043 in which the fixation mechanism 3043 is provided with a roughened or porous surface feature 3062. Surface feature 3062 promotes the ingrowth of collagenous encapsulation tissue ("scar tissue") following implantation of the lead 3036 to enhance long term fixation of the lead 3036 within the heart 12.

According to one embodiment, as shown in FIG. 33B, a rapidly dissolvable coating 3064 is formed over the fixation mechanism 3043. The coating 3064 may be water soluble. Suitable rapidly dissolvable coatings are described above. The coating 3064 masks the surface feature 3062, reducing friction between the surface feature 3062 and the heart 12, facilitating passage of the fixation mechanism 3043 through the heart 12.

The coating 3064 masks the shape of the fixation mechanism 3043 as well as the surface feature 3062. The coating 3064 retains the fixation mechanism 3043 in the first configuration until the coating 3064 dissolves, preventing premature deployment of the fixation mechanism 3043 to the second configuration. Following dissolution, the fixation mechanism 3043 may be deployed to the second configuration and the surface feature 3062 is revealed to permit tissue ingrowth.

According to one embodiment, the coating 3064 forms a first implant friendly shape around the fixation mechanism 3043. The implant friendly shape may be rounded or have a blunt leading edge and is chosen to reduce trauma to the myocardial tissue 3032 during insertion. According to still another embodiment, shown in FIG. 33C, the coating 3064 forms a more pointed shape chosen to facilitate dissection or dilation of the myocardium 32 during insertion.

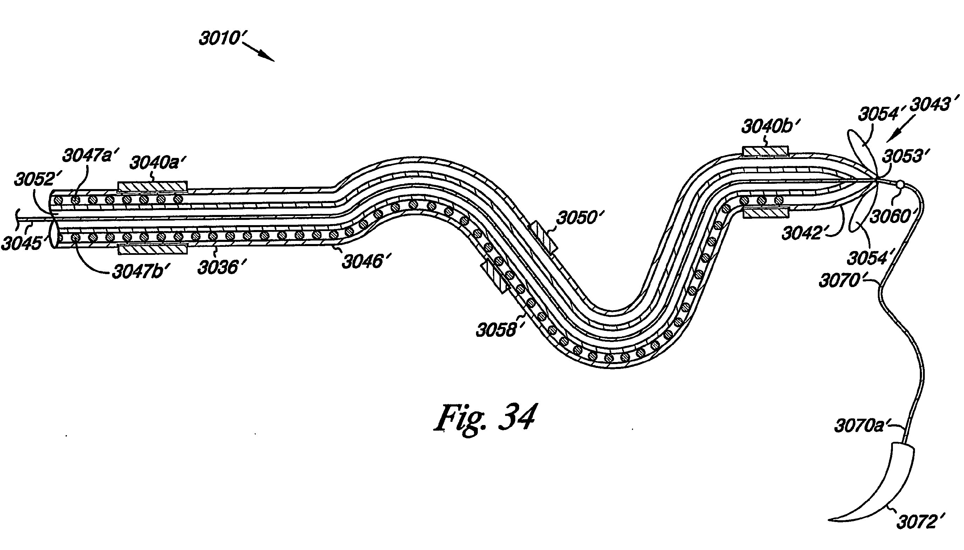

FIG. 34 shows a distal portion of a myocardial lead attachment and pacing system 3010' according to another embodiment of the present invention. System 3010' is generally similar to system 3010 of FIG. 30 and includes many of the same features. Contrary to the embodiment shown in FIG. 30, myocardial lead attachment system 3010' is adapted to be inserted into the heart 12 without the use of a delivery instrument as previously described. According to the embodiment shown in FIG. 34, the myocardial lead 3036' is coupled to a pull-through type suture 3070', which is coupled at a distal end 3070a' to a needle 3072'. The needle 3072' is used to penetrate the epicardial surface 28 and is advanced through the myocardium 32. The lead 3036' is pulled behind the needle 3072' through the heart 12 with the pull-through suture 3070'. The fixation mechanism 3043' is retained in a first configuration as the lead 3036' is advanced through the heart 12. When the distal tip 3042' of the lead 3036' has reached a chosen implant site, the fixation mechanism 3043' is deployed to a second configuration to secure the lead 3036' in place. The suture 3070' and needle 3072' are then cut from the lead 3036 and removed.

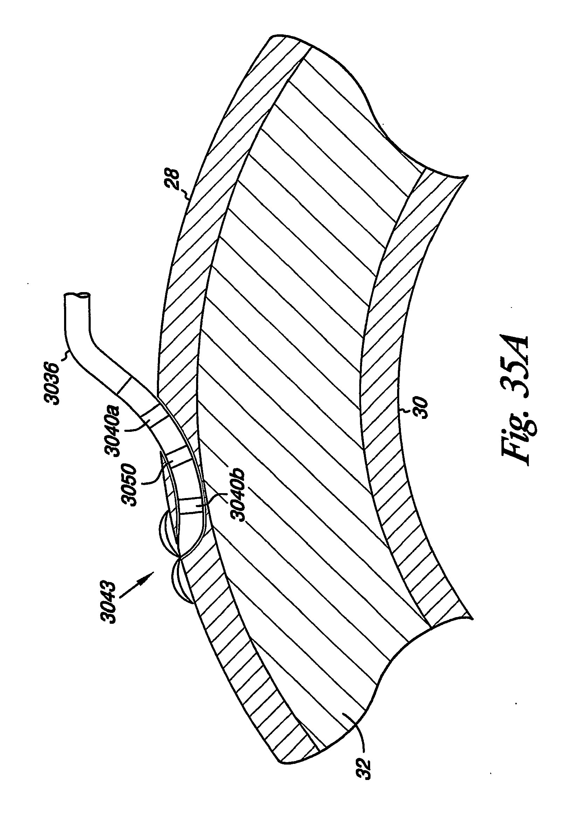

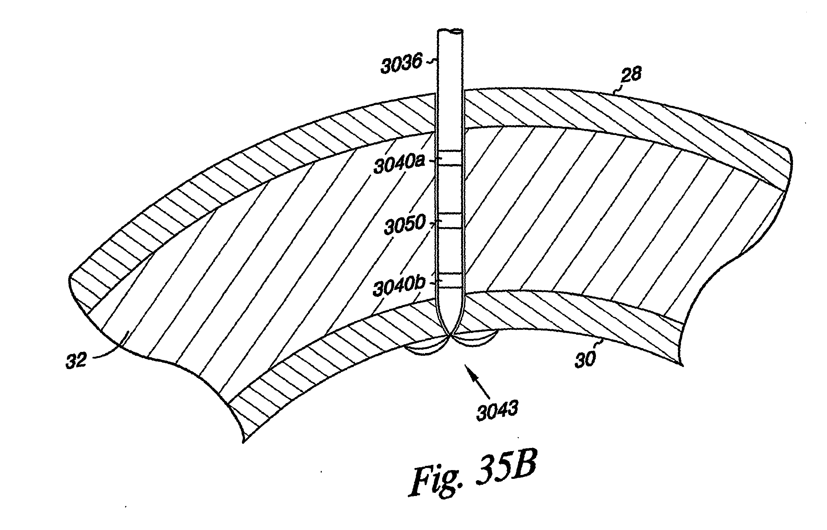

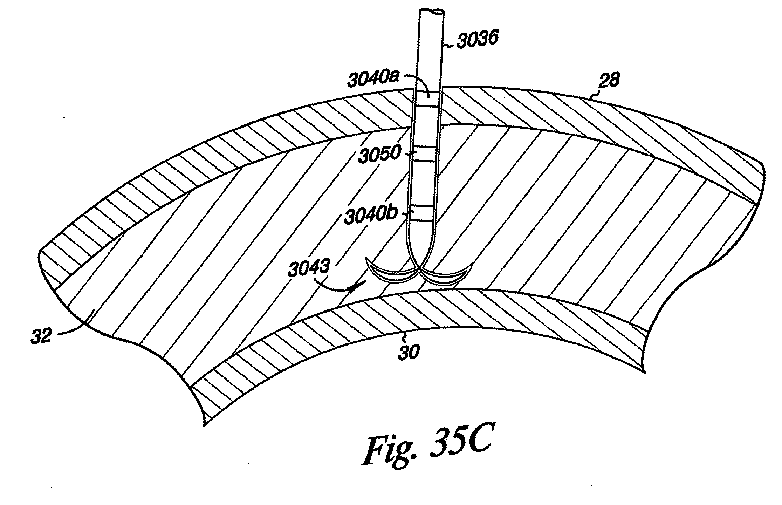

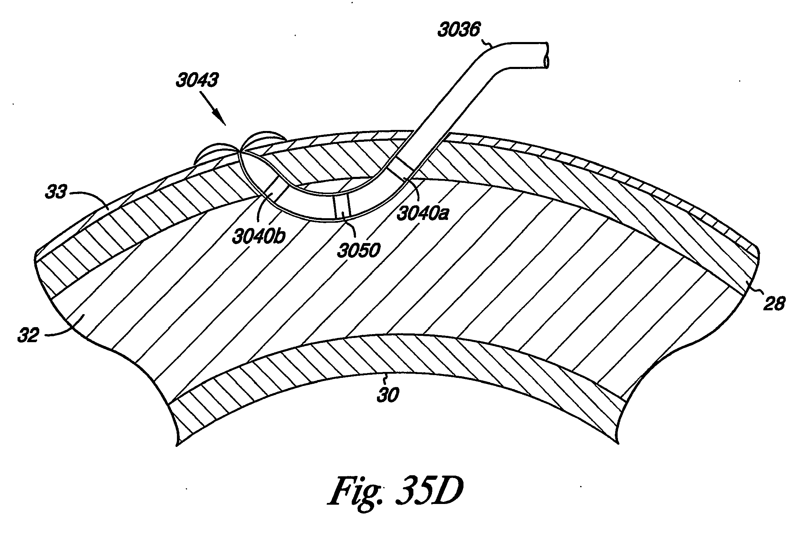

FIGS. 35A - 35D shows sectional views of a portion of the vasculature and a distal portion of the myocardial lead attachment system 3010 of FIG. 30 with the fixation mechanism 3043 deployed in the heart 12 in various lead configurations. The fixation mechanism 3043 may be deployed on a surface of the epicardium 28 (FIG. 35A), the endocardium 30 (FIG. 35B), within the myocardium 32 (FIG. 35C), or on the surface of the pericardium 33 (FIG. 35D). The fixation mechanism 3043 resists proximally directed axial forces exerted on the lead 3036. The lead 3036 is advantageously stabilized and intra-myocardial motion and migrations are reduced. While generally showing the system 3010 of FIG. 30, the system 3010' of FIG. 34 may also be deployed as shown.

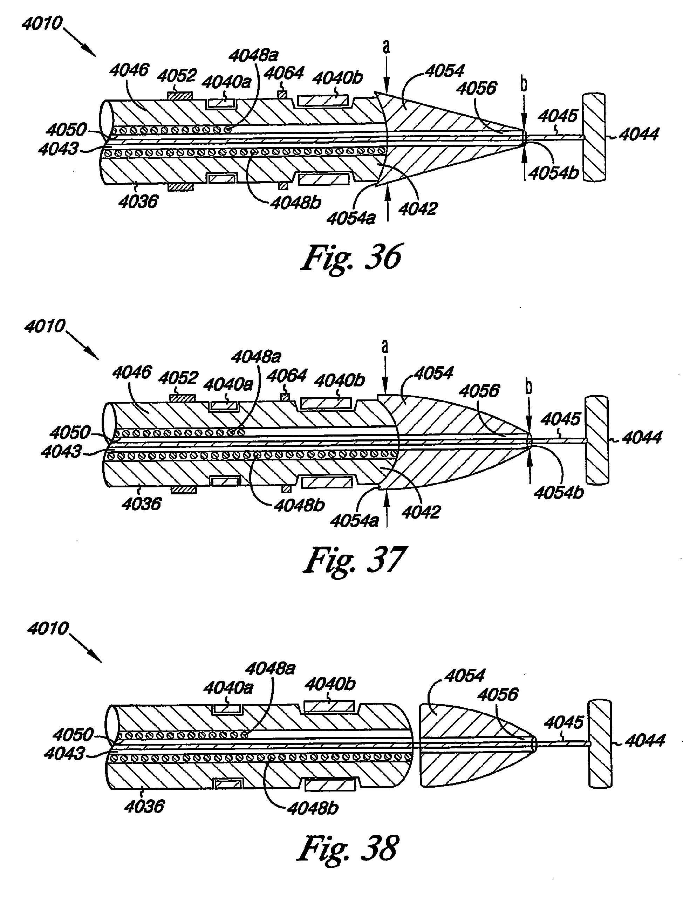

FIG. 36 is a sectional view of a distal portion of a myocardial lead attachment system 4010 according to another embodiment of the present invention. A myocardial lead 4036 includes two electrodes, a proximal anode 4040a and a distal cathode 4040b. An outer insulating sheath 4046 is formed around the lead 4036 and protects a pair of coiled conductive members 4048a and 4048b coupled to the anode 4040a and cathode 4040b, respectively. A second inner insulating sheath 4050 forms an internal lumen 4043 for receiving the tether 4045. A marker band 4052 is optionally formed on the outer insulating sheath 4046.

The lead 4036 includes a tapered tip 4054 positioned distal to the distal region 4042 of the lead 4036. The tapered tip 4054 tapers from a first diameter a at a proximal end 4054a to a second diameter b, smaller than the first diameter a, at a distal end 4054b. In one embodiment, as shown in FIG. 36, the tapered tip 4054 is formed in the shape of a cone. In another embodiment, shown in FIG. 37, the tip 4054 is more rounded and is formed in the shape of a bullet. A bore 4056 extends through the tip 4054 in communication with the lumen 4043 for receiving the tether 4045.

As the lead 4036 is advanced over the tether 4045 through the during insertion, the tapered tip 4054 does not cut through the myocardial tissue 32, but rather dissects or dilates the tissue. The tapered tip 4054 provides a streamlined leading edge to the lead 4036, reducing trauma to the myocardium 32. According to one embodiment, the diameter a of the proximal end 4054a is greater than a diameter of the lead 4036. Such a tip 4054 gently dilates or expand the tract to facilitate advancement of the lead 4036. According to other embodiments, the tip 4054 has any shape having rounded edges and a streamlined shape chosen to reduce trauma to the myocardium 32 during insertion.

Prior to lead implantation, the tip 4054 may be selected from a plurality of tips having differing shapes based on the physiology of the heart 12. Where the epicardium 28 and or pericardium 33 are generally undisturbed and in relatively healthy condition, the more bullet shaped tip of the embodiment shown in FIG. 37 is sufficient to facilitate advancement of the lead 4036. However, sometimes the epicardium 28 and/or pericardium 33 have been damaged, either by disease or previous trauma, resulting in the presence of tough adhesions or scar tissue. The more pointed cone shaped tip 4054 of the embodiment shown in FIG. 36 may be required to effectively traverse such adhesions or scar tissue. Prior to inserting the lead 4036, the surgeon may evaluate the implant site and select an appropriate tip 4054, *i.e.* pointed or blunt, as deemed necessary to pierce the epicardium 28 and or pericardium 33 and dilate the tract through the heart 12 to facilitate insertion of the lead 4036.

According to one embodiment, as shown in FIGS. 36 and 37, the tapered tip 4054 is configured to securely couple with the blunt distal tip 4042 of the lead 4036. According to one embodiment, the diameter a of the proximal end 4054a of the tip 4054 is sized to receive the distal tip 4042 of the lead 4036. According to other embodiments, the tip 4054 and distal tip 4042 of the lead 4036 are provided with complementary threads for rotational coupling, or are provided with a complementary interlock or other structure for coupling.

FIG. 38 shows another embodiment, in which the tapered tip 4054 is positioned adjacent to the distal tip 4042 of the pacing lead 4036 without securely coupling to the lead 4036. The tapered tip 4054 rides along the tether 4045 in front of the lead 4036 to facilitate the lead 4036 in passing through the myocardium 32. A tip 4054 according to the present embodiment may be used in conjunction with any such commercially available myocardial lead. According to another embodiment, the tapered tip 4054 is integrally formed at the distal end 4042 of the lead 4036.

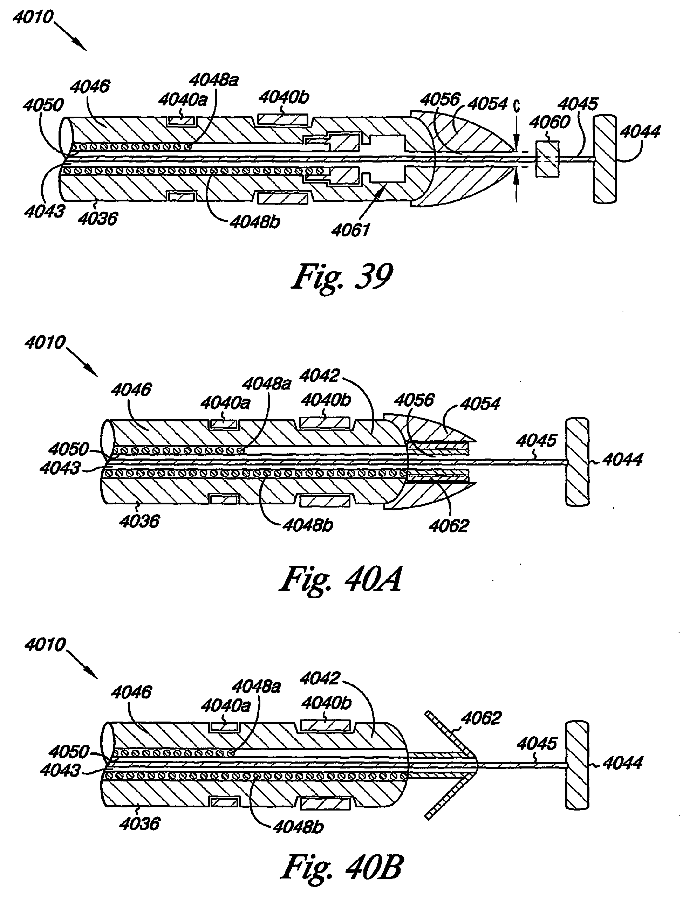

FIG. 39 shows another embodiment in which the system is further provided with a lock 4060 and lock housing 4061 as is described above. The tip bore 4056 has a diameter c greater than the diameter of the tether 4045 such that the tip 4054 easily passes over the tether 4045, but smaller than a diameter of the lock 4060 formed on the tether 4045. The tip 4054 and lead 4036 are easily threaded over the tether 4045 and advanced along the tether 4045. When the tip 4054 contacts the lock 4060, the tip 4054 and lead 4036 are prevented from advancing further along the tether 4045. The tapered tip 4054 is used to prevent the lead 4036 from advancing over the lock 4060, and to provide spacing between the lead 4036 and the anchor mechanism 4044.

According to another embodiment, the tip 4054 is made from a watersoluble material, such that the tip 4054 will dissolve upon placement within the myocardium 32. The tip 4054 may be made from any biocompatible, watersoluble material known in the art, such as a sugar. In one embodiment, the tip 4054 is made from mannitol. In another embodiment, the tip 4054 is made from polyethylene glycol ("PEG"). The molecular weight of the PEG can be selected to achieve a desired dissolution time of the tapered tip 4054. In yet another embodiment, additives known in the art are used to further control the dissolution time. According to another embodiment, the tip 4054 is made of an ablatable material.

A dissolvable tip 4054 reduces the amount of foreign matter located in the heart 12 following dissolution. This may reduce irritation in the heart 12, as well as the formation of scar tissue. Addition of the tip 4054 does not increase the overall size of the lead 4036 chronically implanted in the heart 12. Following dissolution of the tip 4054, the lead 4036 may be advanced over the lock 4060 to mate the lock 4060 with the lock housing 4061. In addition, the dissolved portion of the dissolving tip 4054 provides a lubricating coating or film within the tract to further facilitate passage of the lead 4036.

FIGS. 40A and 40B show another embodiment of the lead 4036, in which a fixation mechanism 4062 is provided at the distal tip 4042 of the lead 4036. Such a fixation mechanism 4062 facilitates fixation of the lead 4036 to myocardial tissue 32. Fixation mechanisms suitable for use with a lead 4036 according to the present embodiment are described above. The tapered tip 4054 is dissolvable as previously described and is configured to mate with the fixation mechanism 4062. According to one embodiment, the fixation mechanism 4062 is received in the tip bore 4056.

Throughout insertion of the lead 4036 into the heart 12, the tapered tip 4054 facilitates passage of the lead 4036 through the tract and masks the fixation mechanism 4062, which may include sharp edges or points. Upon dissolution of the tip 4054, the fixation mechanism 4062 is revealed and operable to retain the lead 4036 in a stable position. According to one embodiment, the fixation mechanism 4062 is retained in the tip bore 4056 in a first collapsed or retracted configuration, as is shown in FIG. 40A. Following dissolution of the tip 4054, the fixation mechanism 4062 deploys to a second expanded configuration, as is shown in FIG. 40B.

According to another embodiment, the tip 4054 contains a pharmaceutical additive to treat implant trauma. Such an additive may be provided to reduce myocardial irritation or inflammation. This pharmaceutical additive may administer a "bolus" therapeutic agent to treat the implant trauma. In one embodiment, the tip 4054 is made of a dissolvable material as described above but which also includes a steroid (or other therapeutic drug) released as the tip 4054 dissolves. According to another embodiment, the tip 4054 is formed of a material provided with a coating that is drug eluting. The tip 4054 may be chosen to have an appropriate amount of steroid (or other therapeutic drug) for a particular situation.

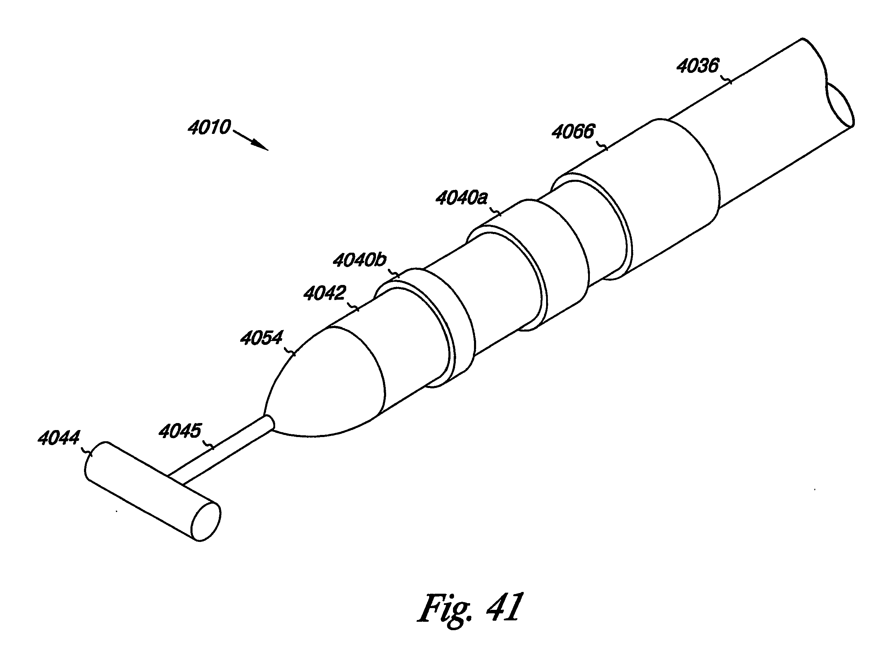

In one embodiment, pharmaceutical additives as previously described are provided on other portions of the lead 4036 in addition to or instead of the tip 4054. In one embodiment, the drug eluting feature is provided as one or more discrete steroid/polymeric rings or collars 4064 positioned on the lead 4036 to contact the myocardium 32 upon implantation and anchoring (See FIG. 36). In another embodiment, the implanted portion of the lead 20 is coated with a drug (e.g., steroid) eluting coating, such as a paint stripe (not shown). In another embodiment, shown in FIG. 41, a polymeric lead body tubing 4066 may be fashioned from a steroid-loaded polymer composite. In each of these embodiments, a therapeutic amount of steroid is included on the implanted portion of the lead 4036.

Various controlled-release techniques known in the art may be incorporated into these embodiments to deliver the therapeutic drug in the right amount and with the right time distribution.

Although the present invention has been described with reference to preferred embodiments, persons skilled in the art will recognize that changes may be made in form and detail without departing from the scope of the invention.

## Claims

1. A myocardial lead attachment system (10) for securing a distal end of a lead within a myocardium (32) of a patient's heart (12), the attachment system (10) having an anchor (44), an anchor extension (45) coupled to the anchor (44), and a delivery instrument (50) for receiving and advancing the anchor (44) through a tract in the heart (12) from a proximal entrance site to a distal exit site such that the anchor extension (45) extends proximally from the anchor (44) through the tract whereby
the delivery instrument (50) comprises a nest (62) positioned proximal to the distal tip (60) of the delivery instrument (60) and sized to receive the anchor (44), wherein the nest (62) includes an elongated slot opening to the needle lumen (59); and
an ejection mechanism (66) for ejecting the anchor (44) from the nest (62), **characterised in that** the anchor extension (45) is a tether and the delivery instrument (50) comprises a needle (58) having a closed distal tip (60).

2. The attachment system of claim 1 wherein the ejection mechanism (66) further comprises an actuator (84) for ejecting the anchor (44) from the nest (62).

3. The attachment system of claim 1 further comprising a lead body (36, 1036, 1102, 1204, 1304, 1402, 2102, 2142, 3036, 4036) halving a proximal end, a distal end and a lumen (1043, 1110, 1214, 2146, 1310, 1410, 2155, 3052, 4043) for accepting the tether (45, 1045, 1112, 1208, 1308, 1412, 2106, 2146, 3045, 4045).

4. The attachment system of claim 3 further comprising a capered tip (4054) separate from the lead body (4036) and positioned adjacent the distal end (4042) of the lead body (4036), wherein the tip (4054) has a longitudinal through hole (4056) for accepting the tether (4045).

5. The attachment system of claim 4 wherein the tapered tip (4054) is formed from a dissolvable material.

6. The attachment system of claim 4 wherein the tapered tip (4054) is formed from a therapeutic drug-eluting material.

7. The attachment system of claim 3 further comprising a fixation mechanism (4062) at the distal end (4042) of the lead body (4036), wherein the fixation mechanism (4062) is adapted to collapse to a first position during implantation and deploy to a second position after implantation.

8. The attachment system of claim 3 wherein either of the tether (45, 1045, 1112, 1208, 1308, 1412, 2106, 2146, 3045, 4045) or the anchor (44) is formed of a bioabsorbable or biodegradable polymer and further comprising a surface feature formed on a portion of the lead body (36, 1036, 1102, 1204, 1304, 1402, 2102, 2142, 3036, 4036) for promoting formation of scar tissue around said portion of the lead body (36, 1036, 1102, 1204, 1304, 1402, 2102, 2142, 3036, 4036).

9. The attachment system of claim 3 further comprising: a lock structure (1048, 4060) on the tether (1045, 4045); and a lock housing (1049, 4061) in the lumen (1110, 4043), wherein the lock structure (1048, 4060) mates with the lock housing (1049, 4061) and restrains motion of the lead body (1036, 4036) with respect to the tether (1045, 4045) in either of a proximal or a distal direction.

10. The attachment system of claim 9 further comprising a proximal lock (1312) for engaging the tether (1308) to the lead body (1304) proximal to the lock housing (1049, 4061), the proximal lock (1312) comprising a second lock structure (1312) formed on the tether (1308) for fixing the tether (1308) in frictional engagement to the proximal end of the lead body (1304).

11. The attachment system of claim 1 wherein the nest (62) includes a tether slot (78) for accepting the tether (45).

12. The attachment system of claim 1 wherein the nest (62) includes a ramp (76) inclined at an angle such that sliding the anchor (44) causes the anchor (44) to ride up the ramp (76) and out of the nest (62).

## Patentansprüche

1. Myokardiales Leitungsbefestigungssystem (10), um ein distales Ende einer Leitung in einem Myoardium (32) von einem Herz (12) eines Patienten zu befestigen, wobei das Befestigungssystem (10) eine Verankerung (44), eine mit der Verankerung (44) gekoppelte Verankerungsverlängerung (45) und ein Zuführinstrument (50) zum Aufnehmen und zum Vorbewegen der Verankerung (44) durch eine Bahn in dem Herz (12) von einer proximalen Eintrittsstelle zu einer distalen Austrittsstelle aufweist, so dass sich die Verankerungsverlängerung (45) proximal von der Verankerung (44) durch die Bahn erstreckt, wobei
das Zuführinstrument (50) eine Aufnahme (62), die proximal zu der distalen Spitze (60) des Zuführinstruments (50) angeordnet und bemessen ist, um die Verankerung (44) aufzunehmen, wobei die Aufnahme (62) einen länglichen Schlitz aufweist, der zum Nadel-Lumen (59) offen ist, und
einen Ausstoßmechanismus (66) zum Ausstoßen der Verankerung (44) aus der Aufnahme (62) aufweist, **dadurch gekennzeichnet, dass**
die Verankerungsverlängerung (45) ein Tether ist und das Zuführinstrument (50) eine Nadel (58) mit einer geschlossenen distalen Spitze (60) aufweist.

2. Befestigungssystem nach Anspruch 1, bei dem der Ausstoßmechanismus (66) außerdem ein Betätigungsmittel (84) zum Ausstoßen der Verankerung (44) aus der Aufnahme (62) aufweist.

3. Befestigungssystem nach Anspruch 1, das außerdem einen Leitungskörper (36, 1036, 1102, 1204, 1304, 1402, 2102, 2142, 3036, 4036) mit einem proximalen Ende, einem distalen Ende und einem Lumen (1043, 1110, 1214, 2146, 1310, 1410, 2155, 3052, 4043) aufweist, um den Tether (45, 1045, 1112, 1208, 1308, 1412, 2106, 2146, 3045, 4045) aufzunehmen.

4. Befestigungssystem nach Anspruch 3, außerdem mit einer konisch zulaufenden Spitze (4054), die von dem Leitungskörper (4036) getrennt und benachbart dem distalen Ende (4042) des Leitungskörpers (4036) angeordnet ist, wobei die Spitze (4054) ein längliches Durchgangsloch (4056) zur Aufnahme des Tethers (4045) hat.

5. Befestigungssystem nach Anspruch 4, bei dem die konisch zulaufende Spitze (4054) aus einem sich auflösenden Material gebildet ist.

6. Befestigungssystem nach Anspruch 4, bei dem die konisch zulaufende Spitze (4054) aus einem ein therapeutisches Arzneimittel eluierenden Material gebildet ist.

7. Befestigungssystem nach Anspruch 3, außerdem mit einem Fixierungsmechanismus (4062) an dem distalen Ende (4042) des Leitungskörpers (4036), wobei der Fixierungsmechanismus (4062) ausgestaltet ist, um während des Implantierens in eine erste Position einzuklappen und um nach dem Implantieren in eine zweite Position auszuklappen.

8. Befestigungssystem nach Anspruch 3, bei dem entweder der Tether (45, 1045, 1112, 1208, 1308, 1412, 2106, 2146, 3045, 4045) oder die Verankerung (44) aus einem bio-absorbierbaren oder bio-degradierbaren Polymer gebildet ist und außerdem ein Oberflächenmerkmal aufweist, das an einem Bereich des Leitungskörpers (36, 1036, 1102, 1204, 1304, 1402, 2102, 2142, 3036, 4036) gebildet ist, um die Ausbildung von Narbengewebe um diesen Bereich des Leitungskörpers (36, 1036, 1102, 1204, 1304, 1402, 2102, 2142, 3036, 4036) zu unterstützen.

9. Befestigungssystem nach Anspruch 3, außerdem mit: eine Arretierungsstruktur (1048, 4060) an dem Tether (1045, 4045); und ein Arretierungsgehäuse (1049, 4061) in dem Lumen (1110, 4043), wobei die Arretierungsstruktur (1048, 4060) mit dem Arretierungsgehäuse (1049, 4061) zusammenpasst und eine Bewegung des Leitungskörpers (1036, 4036) bezüglich des Tethers (1045, 4045) entweder in einer proximalen oder einer distalen Richtung begrenzt.

10. Befestigungssystem nach Anspruch 9, außerdem mit einer proximalen Arretierung (1312), um den Tether (1308) mit dem Leitungskörper (1304) proximal zu dem Arretierungsgehäuse (1049, 4061) in Eingriff zu bringen, wobei die proximale Arretierung (1312) eine zweite Arretierungsstruktur (1312) aufweist, die an dem Tether (1308) ausgebildet ist, um den Tether (1308) in Reibeingriff mit dem proximalen Ende des Leitungskörpers (1304) zu fixieren.

11. Befestigungssystem nach Anspruch 1, bei dem die Aufnahme (62) einen Tether-Schlitz (78) zum Aufnehmen des Tethers (45) aufweist.

12. Befestigungssystem nach Anspruch 1, bei dem die Aufnahme (62) eine Rampe (76) hat, die mit einem Winkel so abgeschrägt ist, dass ein Gleiten der Verankerung (44) bewirkt, dass die Verankerung (44) an der Rampe (76) hoch und aus der Aufnahme (62) heraus gleitet.

## Revendications

1. Système de fixation (10) d'un fil conducteur myocardique permettant de fixer une extrémité distale d'un fil conducteur à l'intérieur du myocarde (32) du coeur d'un patient (12), le système de fixation (10) comportant un dispositif d'ancrage (44), une extension (45) du dispositif d'ancrage couplée au dispositif d'ancrage (44) et un instrument d'administration (50) permettant de réceptionner et de faire progresser le dispositif d'ancrage (44) à travers une zone du coeur (12) depuis un site d'entrée proximal jusqu'à un site de sortie distal, de manière que l'extension du dispositif d'ancrage (45) s'étende de manière proximale du dispositif d'ancrage (44) à travers la zone, moyennant quoi l'instrument d'administration (50) comprend un logement (62) positionné à proximité de l'embout distal (60) de l'instrument d'administration (50) et dimensionné pour réceptionner le dispositif d'ancrage (44), où le logement (62) comporte une ouverture en forme de fente allongée pour la lumière d'aiguille (59), et
un mécanisme d'éjection (66) permettant d'éjecter le dispositif d'ancrage (44) du logement (62), **caractérisé en ce que** l'extension du dispositif d'ancrage (45) est un câble d'attache et l'instrument d'administration (50) comprend une aiguille (58) comportant un embout distal fermé (60).

2. Système de fixation selon la revendication 1, dans lequel le mécanisme d'éjection (66) comprend en outre un dispositif d'actionnement (84) permettant d'éjecter le dispositif d'ancrage (44) du logement (62).

3. Système de fixation selon la revendication 1 comprenant en outre un corps de fil conducteur (36, 1036, 1102, 1204, 1304, 1402, 2102, 2142, 3036, 4036) ayant une extrémité proximale, une extrémité distale et une lumière (1043, 1110, 1214, 2146, 1310, 1410, 2155, 3052, 4043) permettant de réceptionner le câble d'attache (45, 1045, 1112, 1208, 1308, 1412, 2106, 2146, 3045, 4045).

4. Système de fixation selon la revendication 3 comprenant en outre un embout effilé (4054) séparé du corps du fil conducteur (4036) et positionné adjacent à l'extrémité distale (4042) du corps du fil conducteur (4036), dans lequel l'embout (4054) comporte un trou traversant longitudinal (4056) permettant de réceptionner le câble d'attache (4045).

5. Système de fixation selon la revendication 4, dans lequel l'embout effilé (4054) est constitué à partir d'une matière pouvant se dissoudre.

6. Système de fixation selon la revendication 4, dans lequel l'embout effilé (4054) est constitué à partir d'une matière d'élution de médicament thérapeutique.

7. Système de fixation selon la revendication 3 comprenant en outre un mécanisme de fixation (4062) situé au niveau de l'extrémité distale (4042) du corps du fil conducteur (4036), dans lequel le mécanisme de fixation (4062) est conçu pour s'escamoter dans une première position au cours de l'implantation et se déployer dans une seconde position après implantation.

8. Système de fixation selon la revendication 3, dans lequel soit le câble d'attache (45, 1045, 1112, 1208, 1308, 1412, 2106, 2146, 3045, 4045) soit le dispositif d'ancrage (44) est constitué d'un polymère bioabsorbable ou biodégradable et comprenant en outre une caractéristique de surface formée sur une partie du corps du fil conducteur (36, 1036, 1102, 1204, 1304, 1402, 2102, 2142, 3036, 4036) permettant de favoriser une formation de tissu cicatriciel autour de ladite partie du corps du fil conducteur (36, 1036, 1102, 1204, 1304, 1402, 2102, 2142, 3036, 4036).

9. Système de fixation selon la revendication 3 comprenant en outre une structure de verrouillage (1048, 4060) sur le câble d'attache (1045, 4045) et un boîtier de verrouillage (1049, 4061) dans la lumière (1110, 4043), dans lequel la structure de verrouillage (1048, 4060) s'emboîte dans le boîtier de verrouillage (1049, 4061) et limite un déplacement du corps du fil conducteur (1036, 4036) par rapport au câble d'attache (1045, 4045) dans une direction proximale ou dans une direction distale.

10. Système de fixation selon la revendication 9 comprenant en outre un dispositif de verrouillage proximal (1312) permettant de mettre en contact le câble d'attache (1308) et le corps du fil conducteur (1304) à proximité du boîtier de verrouillage (1049, 4061), le dispositif de verrouillage proximal (1312) comprenant une seconde structure de verrouillage formée sur le câble d'attache (1308) permettant de fixer le câble d'attache (1308) en contact de friction sur l'extrémité proximale du corps du fil conducteur (1304).

11. Système de fixation selon la revendication 1, dans lequel le logement (62) comprend une fente pour le câble d'attache (78) permettant de réceptionner le câble d'attache (45).

12. Système de fixation selon la revendication 1, dans lequel le logement (62) comprend une rampe (76) inclinée selon un angle tel qu'un coulissement du dispositif d'ancrage (44) permet au dispositif d'ancrage (44) de monter la rampe (76) et de sortir du logement (62).
